(19)

Europäisches Patentamt

European Patent Office

Office européen des brevets

(11) **EP 4 541 269 A1**

(12) **EUROPEAN PATENT APPLICATION**

(43) Date of publication:
23.04.2025 Bulletin 2025/17

(21) Application number: 23204570.8

(22) Date of filing: **19.10.2023**

(51) International Patent Classification (IPC):
**A61B 5/0205** (2006.01)  **A61B 5/024** (2006.01)
**A61B 5/352** (2021.01)  **A61B 5/00** (2006.01)

(52) Cooperative Patent Classification (CPC):
**A61B 5/7267; A61B 5/0205; A61B 5/02405;**
**A61B 5/352; A61B 5/4812; A61B 5/4818;**
A61B 2562/0204

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **XIE, Jiali**
**Eindhoven (NL)**

• **DOS SANTOS DA FONSECA, Pedro Miguel**
**Ferreira**
**Eindhoven (NL)**
• **LONG, Xi**
**5656AG Eindhoven (NL)**
• **VAN DIJK, Johannes**
**Eindhoven (NL)**
• **OVEREEM, Sebastiaan**
**Eindhoven (NL)**

(74) Representative: **Philips Intellectual Property &**
**Standards**
**High Tech Campus 52**
**5656 AG Eindhoven (NL)**

(54) **AUDIO AND CARDIAC BASED SLEEP-RELATED EVENT DETECTION**

(57)    Disclosed is a method for training a sleep-related event detection model, a corresponding method of sleep-related event detection as well as a corresponding computer program, data structure and data processing device. The training method may comprise a step of providing, as input, a set of training data samples. Each training data sample may comprise one or more cardiac signals representative of a cardiac parameter of a subject, one or more audio recording signals representative of an environmental sound of the subject and at least a first and second sleep-related event of the subject associated with the one or more cardiac and audio recording signals of the subject. The method may comprise a step of training the sleep-related event detection model using the input.

100

```
┌─────────────────────────────────────┐
│ Providing as input, a set of training│
│ data samples, wherein each training  │
│ data sample comprises one or more    │
│ cardiac signals, one or more audio   │
│ recording signals and at least a     │
│ first and second sleep-related event │
│ associated with the one or more      │
│ cardiac and audio recording signals. │
│                 102                  │
└─────────────────────────────────────┘
                  │
                  ▼
┌─────────────────────────────────────┐
│ Training a sleep-related event       │
│ detection model using the input.     │
│                 104                  │
└─────────────────────────────────────┘
```

Fig. 1

EP 4 541 269 A1

**Description**

TECHNICAL FIELD

The present disclosure generally relates to the field of sleep-related event detection, and more particularly to techniques for training a sleep-related event detection model and detecting sleep-related events. Certain embodiments may provide for improved detection accuracy.

BACKGROUND

Sleep-disordered breathing (SDB) in general and obstructive sleep apnea (OSA) in particular are among the most prevalent sleep disorders. It is estimated that, among adults aged between 30 and 70 years, approximately 13% of men and 6% of women exhibit moderate to severe OSA (with an apnea-hypopnea index (AHI) $\geq$ 15), and the incidence of SDB is on the rise worldwide. Currently, the gold standard for diagnosing SDB is a full night polysomnography (PSG). However, PSG has several limitations, such as high cost, substantial labor requirements, patient discomfort due to attached sensors, and long waiting lists, all of which hinder its accessibility. Hence, there is a pressing need for a readily available, cost-effective, and automated approach to monitor SDB for broader public and clinical use.

Various approaches have been studied as alternatives to PSG for SDB event detection and AHI estimation using different signals that are easier to acquire, and/or can be used for longer-term monitoring at home, e.g., to follow up on the effectiveness of OSA therapy.

A recurrent issue observed in the known methods pertains to the AHI estimation, which is calculated as the number of apneas (central or obstructive) plus the number of hypopneas, divided by either the total recording time (TRT) or the total sleep time (TST). When scoring AHI based on PSG, TST can be achieved by simply adding the time of any sleep stage manually scored from the neurological (electroencephalography (EEG), electromyography (EMG), and electrooculography (EOG)) channels recorded with PSG.

However, systems without neurological signals (e.g., polygraphic systems used for home sleep apnea testing) have traditionally relied on TRT to estimate AHI. This is known to introduce underestimation problems, particularly due to a substantial proportion of individuals with SDB also experiencing comorbid insomnia, which reduces the fraction of TST in respect to the TRT.

To alleviate this issue, alternatives described in the literature have used manual sleep annotations derived from PSG for AHI estimation, but this offers disadvantages in actual real-world deployment since it requires the acquisition of the neurophysiological signals typically utilized for sleep staging. This poses challenges in terms of acquisition for home monitoring from a point of view of comfort, and reliability. Moreover, these signals are unlikely to be accessible in alternative sensing setups initially designed to replace PSG.

An alternative is to calculate total sleep time using surrogate methods (e.g., based on cardiorespiratory sensors). The disadvantage of using such a method in parallel to an SDB detection method is three-fold: first, it requires a comparable set of inputs to both tasks. In case these are not the same, then the smallest set of inputs required for such a system must comprise at least all inputs required by the SDB detection method, and all the inputs required by the sleep staging (or sleep-wake detection) method. Second, the computational complexity is increased, since it requires effectively two algorithms to run on the same input before the results can be combined to obtain a more accurate detection of SDB, and an estimation of AHI. Third, the surrogate expression (e.g., in cardiorespiratory activity) of SDB events may be more or less salient depending on which sleep stage it occurs. For example during N3, the changes in e.g. respiratory effort, and sudden changes in heart rate, are much more visible than during REM sleep, which is characterized by constant changes and oscillations in these parameters.

It is therefore an objective of the present disclosure to present a novel approach for sleep-related event detection, thereby overcoming the above-mentioned disadvantages of the prior art at least in part.

SUMMARY OF THE DISCLOSURE

The objective is solved by the subject-matter defined in the independent claims. Advantageous modifications of embodiments of the present disclosure are defined in the dependent claims as well as in the description and the figures.

One aspect of the present disclosure relates to a computer-implemented method of training a sleep-related event detection model. The method may comprise a step of providing, as input, a set of training data samples. Each training data sample may comprise one or more cardiac signals representative of a cardiac parameter of a subject, one or more audio recording signals representative of an environmental sound of the subject and/or at least a first and second sleep-related event of the subject associated with the one or more cardiac and audio recording signals of the subject. The method may comprise a step of training the sleep-related event detection model using the input.

Another aspect of the present disclosure relates to a method of sleep-related event detection of a subject. The method may comprise a step of providing a trained sleep-related event detection model, preferably trained in accordance with the aspects described herein. The method may comprise a step of providing, as input to the sleep-related event detection model, one or more cardiac signals representative of a cardiac parameter of a subject and/or one or more audio recording signals representative of an environmental sound of the subject. The method may comprise a step of detecting, by the sleep-related event detection model, at least a first and/or second sleep-related event of the subject based at least in part on the input to generate a detection result. The method may comprise a step of providing the detection result.

In general, using different data from different sensors (e.g., cardiac and audio recording signals) for training the model may provide a more comprehensive data representation of monitored subjects resulting in an improved detection accuracy of the model compared to models of the prior art. This advantageous data representation also enables to train the model to detect several (i.e., one or more) sleep-related events (i.e., a multi-task model) with improved detection accuracy. The improved detection accuracy is a result of one or more of the following advantages underlying the method(s) of the present disclosure. Different sensors capture different aspects which may result in rich and complementary Information available for training. While cardiac signals may cover certain sleep-related event characteristics, audio recording signals may cover other characteristics. Combining both may result in a complementary and more comprehensive set of information, which comprises a more detailed and accurate representation of the underlying phenomena. Using this representation, the sleep-related detection model is able to extract more informative and discriminative features, leading to better performance. Finally, utilizing cardiac signals, which can be obtained by a single cardiac sensor, and audio recording signals, which are an easily accessible data source, ensures scalability and convenient deployment.

According to another aspect of the present disclosure, the sleep-related event detection model may comprise a shared part configured to generate one or more common features of the at least first and second sleep-related event using the one or more cardiac and/or audio recording signals. The sleep-related event detection model may comprise at least two sleep-related event specific parts. The at least two sleep-related event specific parts may comprise a first sleep-related event specific part configured to generate a detection result for the first sleep-related event and/or a second sleep-related event specific part configured to generate a detection result for the second sleep-related event.

This way an advantageous architecture comprising a shared part and task-specific (i.e., sleep-related event specific) parts is provided which on the one hand is able to learn and generate common features for the sleep-related events from the cardiac and audio recording signals while at the same time being able to learn and generate very task-specific features based on which an improved and simultaneous detection of sleep-related events is achieved.

According to another aspect of the present disclosure, each sleep-related event specific part may further be configured to receive the one or more common features and to generate the detection result of the sleep-related event based at least in part on the one or more common features. It may also be possible that only some of the sleep-related event specific parts receive the one or more common features. It may also be possible that different sleep-related specific parts receive different common features.

Depending on the sleep-related event which is to be detected by the corresponding sleep-related event specific part, using one or more common features generated from the shared part may be reasonable to provide an improved detection result for the corresponding sleep-related event detection.

According to another aspect of the present disclosure, the method(s) may comprise a step of applying, before providing the input, a convolutional neural network (CNN) on the one or more audio recording signals to obtain additional environmental information. The method(s) may comprise a step of providing, as part of the input, the additional environmental information.

By applying a CCN, additional environmental information using the one or more audio recording signals may be obtained. It was found that providing this additional environmental information either additionally or alternatively to the one or more audio recording signals, allows the sleep-related event detection model to better learn and generate meaningful features for the generation of the corresponding detection results. Accordingly, adding the usage of a CNN to the method results in an improved detection accuracy of the model as shown below.

According to another aspect of the present disclosure, the shared part may be using only the one or more cardiac signals. The sleep-related event detection model may comprise a further part configured to generate environmental features based on the one or more audio recordings. The further part may comprise a convolutional recurrent neural network (CRNN). The second sleep-related event specific part may further be configured to generate the detection result of the second sleep-related event based on the environmental features.

It was found that in a situation in which the first sleep-related event (e.g., sleep stage detection also referred to as sleep staging) differs strongly from the second sleep-related event (e.g., SDB event detection), the information underlying the one or more audio recording signals, when being fed into the shared part of the model, may not or at least not significantly improve the model's performance with respect to the detection of the first-sleep related event. Accordingly, to implement the training of the model in a resource efficient manner, the shared part may use only the cardiac signals to generate the common features, which in this case may be referred to as cardiac features. However, in order to be able to utilize the one or more audio recording signals, the model may comprise the further part to generate environmental features based thereon. Subsequently, the first sleep-related event specific part may generate a detection result of the first sleep-related event based on the common/cardiac features. The second sleep-related event specific part may utilize both, i.e., the common/cardiac features and the environmental features to generate the detection result for the second sleep-related event. In other words, it was found that for some use cases such as sleep staging and SDB event detection, sleep staging may be performed with high accuracy only based on features generated from the shared part based on the one or more cardiac signals. Contrary, the performance of SDB event detection may be further improved when combining both signals. Accordingly, this implementation provides a trade-off between resource and model efficiency.

According to another aspect of the present disclosure, the sleep-related detection model may comprise a first part configured to generate a detection result for the first sleep-related event and a preliminary detection result for the second sleep-related event using the one or more cardiac signals. The sleep-related detection model may comprise a second part configured to generate a preliminary detection result for the second sleep-related event using the one or more audio recording signals. The sleep-related detection model may comprise a third part configured to generate a detection result for the second sleep-related event based on the preliminary detection results.

Combining predictions of multiple (sub-)models (i.e., combining the preliminary detections result for the second sleep-related event by the first part and the second part using the third part) improves the overall detection performance of the sleep-related event detection model. Said combination may also be referred to "ensemble learning" or "ensembling". The general idea behind ensembling is to leverage the diversity of multiple models to produce more accurate and robust predictions compared to a single individual model. In addition, by combining multiple models, complexity and capacity issues of single (large) models are avoided.

According to another aspect of the present disclosure, the third part may further be configured to generate a weighting factor. Generating the detection result for the second sleep-related event may further be based on the weighting factor.

The weighting factor allows a flexible combination of the preliminary detection results. For example, the weighting factor may be implemented as a simple binary factor wherein either the preliminary result of the first part or the preliminary result of the second part is taken as final detection result. Alternatively, the weighting factor may also comprise probabilities or the like which allow for a partial consideration of both preliminary results.

According to another aspect of the present disclosure, the first sleep-related event may comprise one or more sleep stages, such as a waking stage and a sleep stage. The second sleep-related event may comprise a sleep disorder breathing (SBD) event. Sleep stages may further comprise wake, N1, N2, N3, and Rapid Eye Movement (REM) or any combination thereof such as combined N1 and N2, combined N1-N3 (also called non-REM).

The term "sleep stage" may be understood as a score assigned (e.g., by a human or machine) to an epoch of predefined duration (e.g., 30 seconds). In general, a sleep stage can be used to designate an epoch as a period in which a subject is either asleep (also referred to as "sleep stage" throughout this disclosure) or awake (also referred to as "waking" or "wake" stage throughout this disclosure). From a neurological point of view, awake may relate to full or partial consciousness. As such, the "wake" stage may comprise one or more consecutive epochs of a state defined in a spectrum ranging from "stages of full awareness and alertness" (also called wakefulness) to "stages of drowsiness" to "stages of unawareness" (i.e., where a subject has no recollection of being awake). Each of these stages may represent very specific neurological characteristics (e.g., using EEG, EOG and/or EMG scoring). These neurological characteristics may comprise presence of alpha rhythm, slow eye movements or other eye movements indicate of some activity (e.g., reading), eye blinks, substantial increase in submental muscle tone (e.g., chin). Contrary to that, a sleep stage may correspond to times when a subject is at sleep, wherein the sleep stage can further be divided into the non-REM stages (N1, N2 and N3) and the REM stage. N1 (also called Non-REM Stage 1) may relate to a transition stage between wake and deeper sleep (e.g., REM). It may be characterized by light sleep and may include drifting off. N2 (also called Non-Rem Stage 2) may relate to a stage of light sleep where eye movement ceases and brain wave patterns become more rhythmic. N3 (also called Non-REM Stage 3) may relate to the deepest stage of Non-REM sleep (also called slow-wave sleep). It may be characterized by slow brain waves. REM stage may be characterized by rapid eye movements, vivid dreams and increase brain activity.

According to another aspect of the present disclosure, the one or more cardiac signals may include electrocardiography (ECG) signals, in particular ECG-derived respiration (EDR) signals and/or signals of respiratory effort. The one or more audio recording signals may comprise Mel Frequency Cepstral Coefficients (MFCCs) extracted from the one or more audio recording signals and/or spectrograms extracted from the one or more audio recording signals.

Implementing MFCCs is an efficient way of extracting efficient features from the audio recording signals, because they are able to capture the relevant spectral characteristics of the audio recording signals. As the MFCCs provide a more compact, yet informative representation of the one or more audio signals, resource efficiency for training and inference is improved while achieving improved detection performance. For example, one audio recording signal may be represented by 12, 13 or another amount of coefficients which represents a significant reduction of required computations.

According to another aspect of the present disclosure, the one or more cardiac signals may be captured using a first sensor means. The first sensor means may be in contact with the subject, in particular a photoplethysmography or a chest-worn accelerometer. The one or more audio recording signals may be captured using a second sensor means such as a microphone, a smartphone and/or a smartwatch. The second sensor means may not be in contact with the subject and/or may be located near to the subject.

This way, reliable and accurate collection of the cardiac and/or audio recording signals is ensured. Placing the first sensor means to be in contact with the subject enables reliable and accurate collection of cardiac signals. On the other hand, it may be sufficient to place the second sensor means near to the subject to collect audio recording signals reliable and accurate. This way, potential interferences between the sensor means can be avoided.

According to another aspect of the present disclosure, the one or more audio recording signals may serve as a surrogate for measured respiratory signals of the subject.

On the one hand, audio signals themselves have long been considered to be inadequate for measuring a subject's breathing activity, but instead only suitable for measuring snoring, silence, and the like. Therefore, it has been accepted among skilled practitioners that audio is generally not a suitable surrogate for respiration. On the other hand, measuring only cardiac signals has long been considered equally inaccurate. The inventors, however, have found out that a machine-learning model can indeed learn from these inputs, and thus surprisingly observed that the selection of input signals according to the aspects disclosed herein is indeed beneficial.

According to another aspect of the present disclosure, a sampling rate of the one or more cardiac signals may be different from a sampling rate of the one or more audio recording signals.

Using different sampling rates for cardiac signals compared to audio recording signals provides flexibility in terms of analyzing the data separately or simultaneously, depending on the specific application at hand. For example, you could analyze the heartbeat using a higher resolution than the audio recordings if you are interested specifically in detecting subtle changes in the cardiac signal that may not be apparent from the lower-resolution audio signals alone.

Another aspect of the present disclosure relates to a data-processing device comprising means for performing the method of any one of the aspects described herein.

Another aspect of the present disclosure relates to a computer program or a computer-readable medium having stored thereon a computer program, the computer program comprising instructions which, when the program is executed by a computer and/or a computer network, cause the computer and/or the computer network to carry out the method of any one of the aspects described herein.

Another aspect of the present disclosure relates to data structure comprising a trained sleep-related event detection model trained using the method of any one of the aspects described herein. The method(s), the data processing device, the computer program or computer readable medium as well as the data structure may find application for example in Sleep/OSA/SDB monitor applications on smartphones based on signals collected from wearable device, wearable devices for OSA/sleep monitoring or any application related to estimation of AHI, OSA severity or sleep disorders.

BRIEF DESCRIPTION OF THE DRAWINGS

The disclosure may be better understood by reference to the following drawings:

Fig. 1: A flowchart of a method for training a sleep-related event detection model in accordance with embodiments of the present disclosure.

Fig. 2: A flowchart of a method of sleep-related event detection in accordance with embodiments of the present disclosure.

Fig. 3: An overview a baseline sleep-related event detection model in accordance with embodiments of the present disclosure.

Fig. 4: First detection results of the baseline sleep-related event detection model in accordance with embodiments of the present disclosure.

Fig. 5: Second detection results of the baseline sleep-related event detection model in accordance with embodiments of the present disclosure.

Fig. 6: Third detection results of the baseline sleep-related event detection model in accordance with embodiments of the present disclosure.

Fig. 7: Three exemplary architectures for the sleep-related event detection model in accordance with embodiments of the present disclosure.

Fig. 8: First detection results of a first sleep-related event detection model in accordance with embodiments of the present disclosure.

Fig. 9: Second detection results of the first sleep-related event detection model in accordance with embodiments of the present disclosure.

Fig. 10: Third detection results of the first sleep-related event detection model in accordance with embodiments of the present disclosure.

Fig. 11: First detection results of a second sleep-related event detection model in accordance with embodiments of the present disclosure.

Fig. 12: Second detection results of the second sleep-related event detection model in accordance with embodiments of the present disclosure.

Fig. 13: Third detection results of the second sleep-related event detection model in accordance with embodiments of the present disclosure.

Fig. 14: First detection results of a third sleep-related event detection model in accordance with embodiments of the present disclosure.

Fig. 15: Second detection results of the third sleep-related event detection model in accordance with embodiments of the present disclosure.

Fig. 16: Third detection results of the third sleep-related event detection model in accordance with embodiments of the present disclosure.

Fig. 17: An overview of a multi-task model in accordance with embodiments of the present disclosure.

Fig. 18: An exemplary implementation of a multi-task model for detecting sleep-related events in accordance with embodiments of the present disclosure.

Fig. 19: First detection results of the multi-task model for detecting sleep-related events in accordance with embodiments of the present disclosure.

Fig. 20: Second detection results of the multi-task model for detecting sleep-related events in accordance with embodiments of the present disclosure.

Fig. 21: Third detection results of the multi-task model for detecting sleep-related events in accordance with embodiments of the present disclosure.

Fig. 22: A performance comparison between the multi-task model and a single task model.

## DETAILED DESCRIPTION

In the following, representative embodiments illustrated in the accompanying drawings will be explained. It should be understood that the illustrated embodiments and the following descriptions refer to examples which are not intended to limit the embodiments to one preferred embodiment.

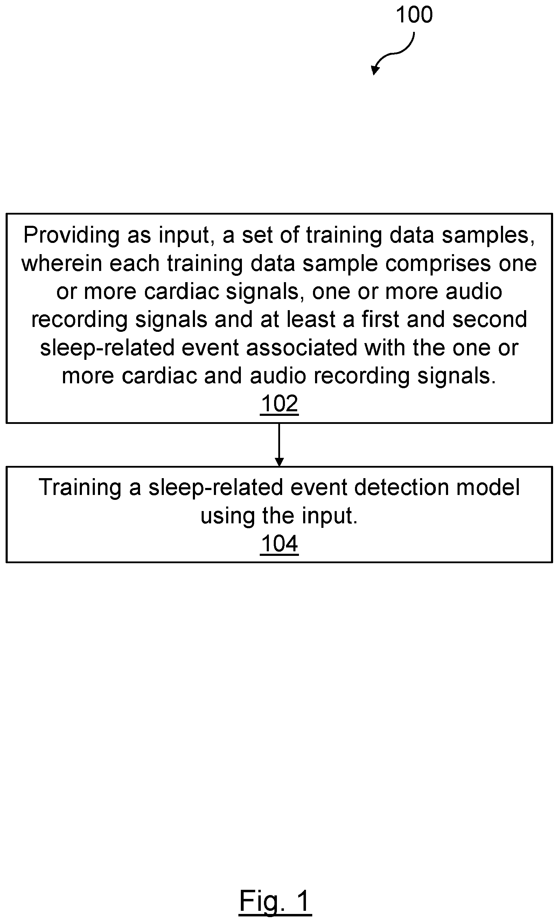

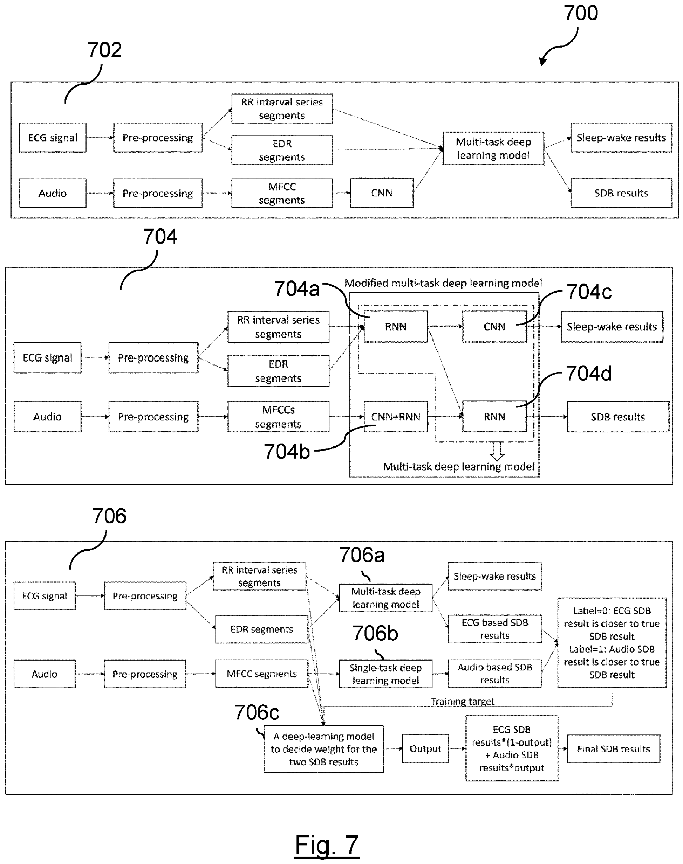

Fig. 1 illustrates a flowchart of a method 100 for training a sleep-related event detection model in accordance with an exemplary embodiment. The sleep-related event detection model may be configured according to the aspects of the present disclosure as for example explained with respect to Figs. 7, 17 and/or 18.

The method 100 may comprise a step 102 of providing, as input, a set of training data samples. Each training data sample may comprise one or more cardiac signals representative of a cardiac parameter of a subject, one or more audio recording signals representative of an environmental sound of the subject and at least a first and second sleep-related event of the subject associated with the one or more cardiac and audio recording signals of the subject.

The method 100 may comprise a step 104 of training the sleep-related event detection model using the input.

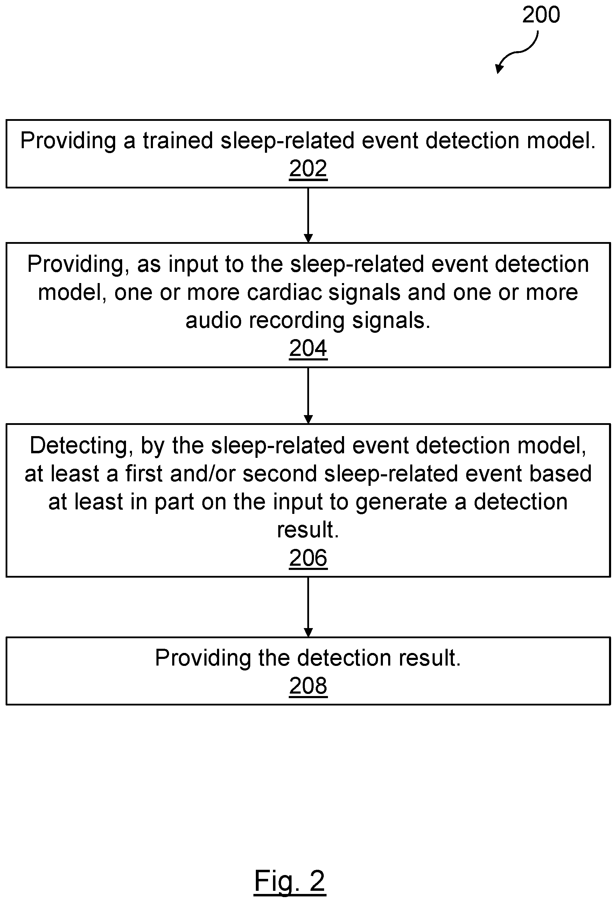

Fig. 2 illustrates a flowchart of a method 200 of sleep-related event detection in accordance with an exemplary embodiment.

The method 200 may comprise a step 202 of providing a trained sleep-related event detection model trained in accordance with the aspects described herein. For example, the sleep-related event detection model may be trained using the training method 100. In this case, it is assumed that the sleep-related event detection model used in method 200 is configured in the same way as the sleep-related event detection model of method 100. The model configuration may be in accordance with the aspects of the present disclosure as for example explained with respect to Figs. 7, 17 and/or 18.

The method 200 may comprise a step 204 of providing, as input to the sleep-related event detection model, one or more cardiac signals representative of a cardiac parameter of a subject and one or more audio recording signals representative of an environmental sound of the subject.

The method 200 may comprise a step 206 of detecting, by the sleep-related event detection model, at least a first and/or second sleep-related event of the subject based at least in part on the input to generate a detection result.

The method 200 may comprise a step 208 of providing the detection result.

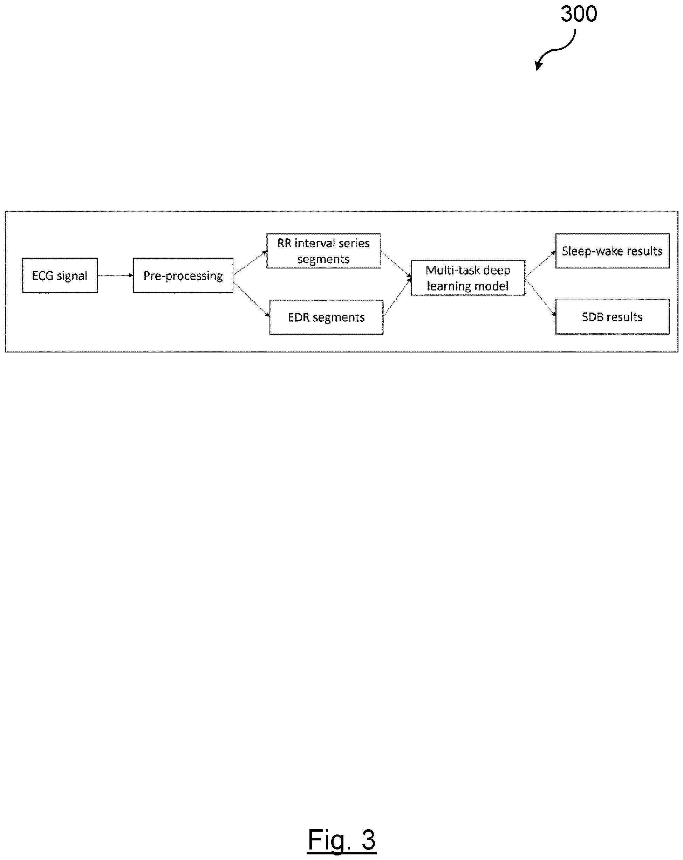

Fig. 3 illustrates an overview of a baseline sleep-related event detection model 300 in accordance with embodiments of the present disclosure. The model 300 may is used as a reference or baseline model for comparing, illustrating and proving the advantages achieved by the sleep-related event detection models configured and trained in accordance with the other aspects of the present disclosure.

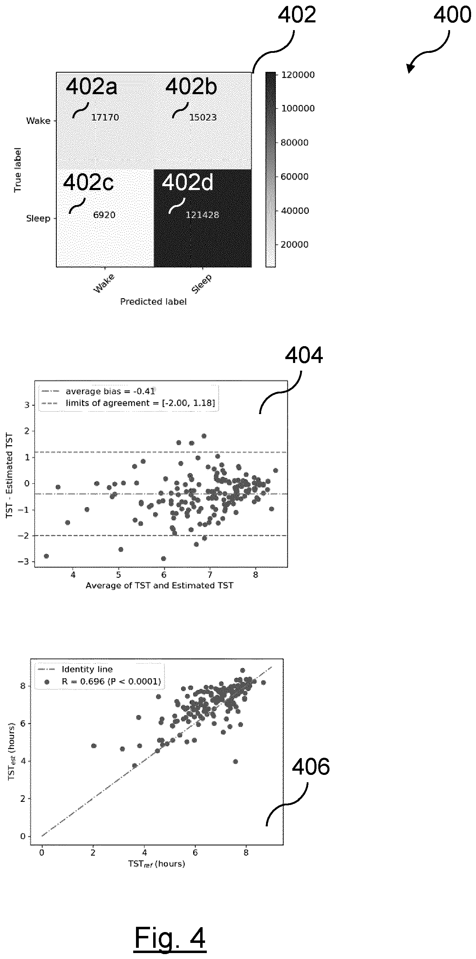

The baseline model 300 is a multi-task deep learning model which only uses cardiac input (e.g., ECG). The collected signals were collected using only a single sensor. By applying corresponding pre-processing, RR-interval series segments as well as ECG-derived respiration (EDR) segments can be extracted from the collected ECG signals. Based on this data, the model 300 was trained to detect SBD events (e.g., a first sleep-related event) in addition to sleep-wake classification (e.g., a second sleep-related event). It should be noted that although a single sensor was used in this baseline model, ECG was pre-processed to obtain a surrogate measure of respiratory effort, namely the EDR segments. Obviously if one would use a cardiac sensor which would not allow for a surrogate measure of respiratory effort, the performance of the baseline model 300 (as explained with respect to Figs.4-6) would be even lower. Fig. 4 illustrates first detection results 400 of the baseline sleep-related event detection model 300. The first detection results 400 relate to sleep-wake detection results based on cardiac input. Confusion matrix 402 illustrates the performance of the baseline model 300. Confusion matrix 402 comprises four fields, which relate to True Positive (TP) detections 402a, True negative (TN) detections 420d, False Positive (FP) detections 402b and False Negative (FN) detections 402c. TPs are instances that were correctly predicted as belonging to the positive class. TNs are instances that were correctly predicted as belonging to the positive class. FPs are instances that were incorrectly predicted as belonging to the positive class. FNs are instances that were incorrectly predicted as belonging to the negative class. As can be seen, the baseline model 300 achieves a solid performance with respect to "sleep" detections. The baseline model 300 detected from a total of 128,348 "sleep" samples, 121,429 samples correctly as "sleep" and only 6,920 wrongly as "wake". However, with respect to "wake" detections, the model 300 achieves an insufficient performance. From 32,193 "wake" samples, the base line model 300 only detected 17,170 correct and 15,023 wrong.

Bland-Altman plot 404 illustrates the total sleep time (TST) estimation results achieved by the model 300. The Bland-Altman plot displays the differences between two measurement methods (e.g., the actual TST and the estimated TST) on the y-axis (vertical axis) against the means/averages of the two methods on the x-axis (horizontal axis). The primary purpose of a Bland-Altman plot is to visualize the level of agreement or bias between the two measurement methods, helping to identify systematic differences, outliers, and the overall spread or variability of the differences. As can be seen, average bias between the actual TST and the estimated TST is -0.41. In other words, this implies that the difference between the TST and the estimated TST is in average negative, i.e., the actual TST is shorter than the estimated TST.

Scatter plot 406 illustrates the estimated TST (in hours) compared to the actual/reference TST (in hours). The "identity line" serves as a reference line that represents a perfect positive linear correlation between the two variables (i.e., $TST_{est}$ and $TST_{ref}$). The Pearson correlation coefficient "R" is a measure of the linear relationship or correlation between the two. R takes values between -1 and 1 and helps to quantify the strength and direction of the linear relationship. R=1 implies a perfect positive linear correlation. In other words, as one variable increases, the other also increases in a linear fashion. R=-1 implies a perfect negative linear correlation. In other words, as one variable increases, the other decreases in a linear fashion. R0= implies no linear correlation. In other words, there is no linear relationship between the two variables. As can be seen, $TST_{est}$ and $TST_{ref}$ are positive linear correlated (see R= 0.696). Ideally, the predictions (visualized as the points) are close to the ideal line. However, as some of the points are still far away from said ideal line, one can follow that the performance of the model 300 can still be improved.

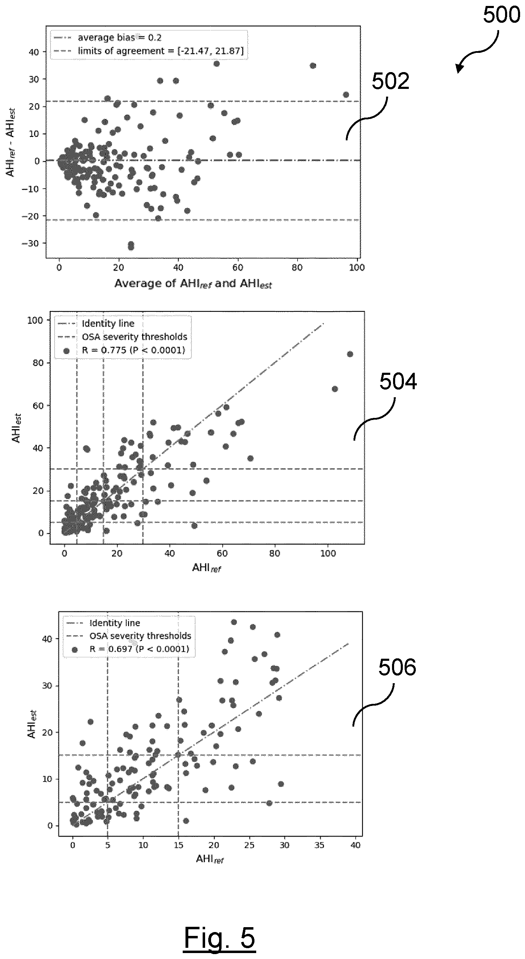

Fig. 5 illustrates second detection results 500 of the baseline sleep-related event detection model 300. The second detection results 500 relate to estimation of apnea-hypopnea index (AHI) based on cardiac input.

Bland-Altman plot 502 illustrates the AHI estimation results achieved by the model 300. The Bland-Altman plot displays the differences between two measurement methods (e.g., the actual/reference $AHI_{ref}$ and the estimated $AHI_{est}$) on the y-axis (vertical axis) against the means/averages of the two on the x-axis (horizontal axis). The primary purpose of a Bland-Altman plot is to visualize the level of agreement or bias between the two measurement methods, helping to identify systematic differences, outliers, and the overall spread or variability of the differences. As can be seen, average bias between the $AHI_{ref}$ and the $AHI_{est}$ is 0.2. In other words, this implies that the difference between the two is in average positive, i.e., the actual AHI is higher than the estimated AHI.

Scatter plot 504 illustrates the estimated AHI compared to the actual/reference AHI. Scatter plot 504 illustrates the estimation results over the entire AHI range. The obstructive sleep apnea (OSA) severity thresholds are indicated by the dotted lines. The "identity line" serves as a reference line that represents a perfect positive linear correlation between the two variables (i.e., $AHI_{ref}$ and the $AHI_{est}$). The Pearson correlation coefficient "R" is a measure of the linear relationship or correlation between the two. R takes values between -1 and 1 and helps to quantify the strength and direction of the linear relationship. R=1 implies a perfect positive linear correlation. In other words, as one variable increases, the other also increases in a linear fashion. R=-1 implies a perfect negative linear correlation. In other words, as one variable increases, the other decreases in a linear fashion. R0= implies no linear correlation. In other words, there is no linear relationship between the two variables. As can be seen, $AHI_{ref}$ and $AHI_{est}$ are positive linear correlated (see R= 0.775). Ideally, the predictions (visualized as the points) are close to the ideal line. However, as some of the points are still far away from said ideal line, one can follow that the performance of the model 300 can still be improved.

Scatter plot 506 illustrates the estimated AHI compared to the actual/reference AHI. Scatter plot 506 only comprises the estimations below the threshold of severe OSA (e.g., $AHI_{ref}$<30). The obstructive sleep apnea (OSA) severity thresholds are indicated by the dotted lines. The "identity line" serves as a reference line that represents a perfect positive linear correlation between the two variables (i.e., $AHI_{ref}$ and the $AHI_{est}$). As can be seen, $AHI_{ref}$ and $AHI_{est}$ are positive linear correlated (see R= 0.697). Ideally, the predictions (visualized as the points) are close to the ideal line. However, as some of the points are still far away from said ideal line, one can follow that the performance of the model 300 can still be improved.

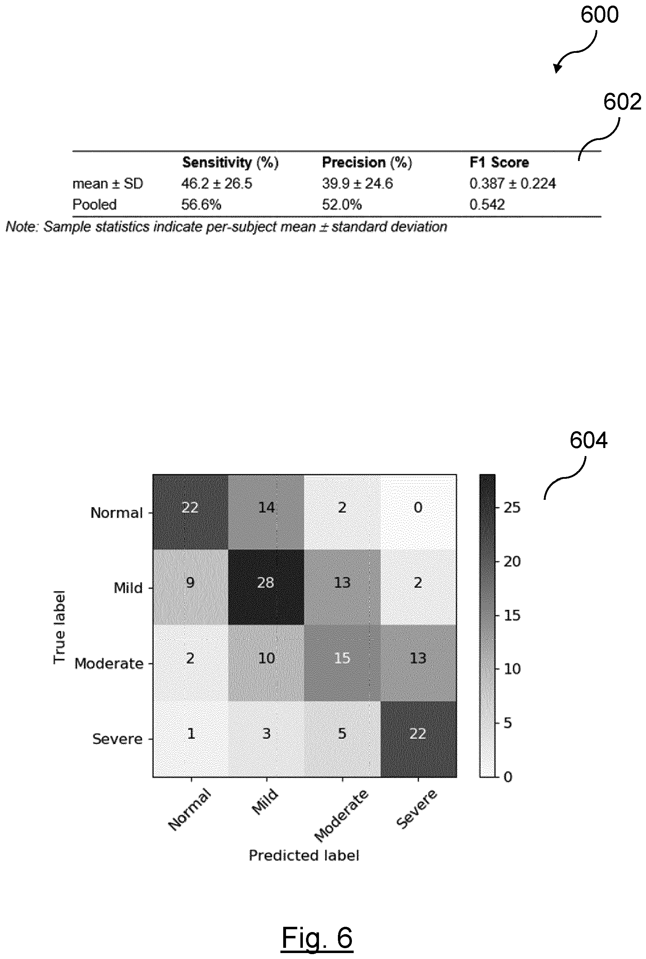

Fig. 6 illustrates third detection results 600 of the baseline sleep-related event detection model 300. The third detection results 600 relate to Sleep-disordered breathing (SBD) detection results based on cardiac input and obstructive sleep apnea (OSA) severity classification results based on cardiac input.

The SBD detection results are illustrated in table 602. The illustrated sample statistics indicate per-subject mean $\pm$ standard deviation (SD). The first column of table 602 comprises the mean $\pm$ standard deviation (SD) sensitivity (%) of 46.2 $\pm$ 26.5 and the pooled sensitivity of 56.6. Sensitivity (or also called Recall or True Positive Rate) indicates the rate between $\frac{TPs}{(TPs+FNs)}$. The second column of table 602 comprises the mean $\pm$ SD precision (%) of 39.9 $\pm$ 24.6 and the pooled precision of 52.0. The precision indicates the rate between $\frac{TPs}{(TPs+FPs)}$. The third column of table 602 comprises the mean $\pm$ SD F1 Score of 0.387 $\pm$ 0.224 and the pooled F1 Score of 0.552. The F1 Score indicates the rate between $2 * \frac{(Precision*Sensitivity)}{(Precision+Sensitivity)}$.

The OSA severity classification results are illustrated in confusion matrix 604. The Confusion matrix illustrates the performance of the baseline model 300 with respect to True Positive (TP) detections, True negative (TN) detections, False Positive (FP) detections and False Negative (FN) detections. The four severity classes the model had to distinguish between are "normal", "mild", "moderate" and "severe" OSA severity. As can be seen, the model 300 performed pretty inaccurate with respect to the detection of "moderate" OSA severity. Furthermore, it can be observed that the model 300 tends to underestimate the OSA severity.

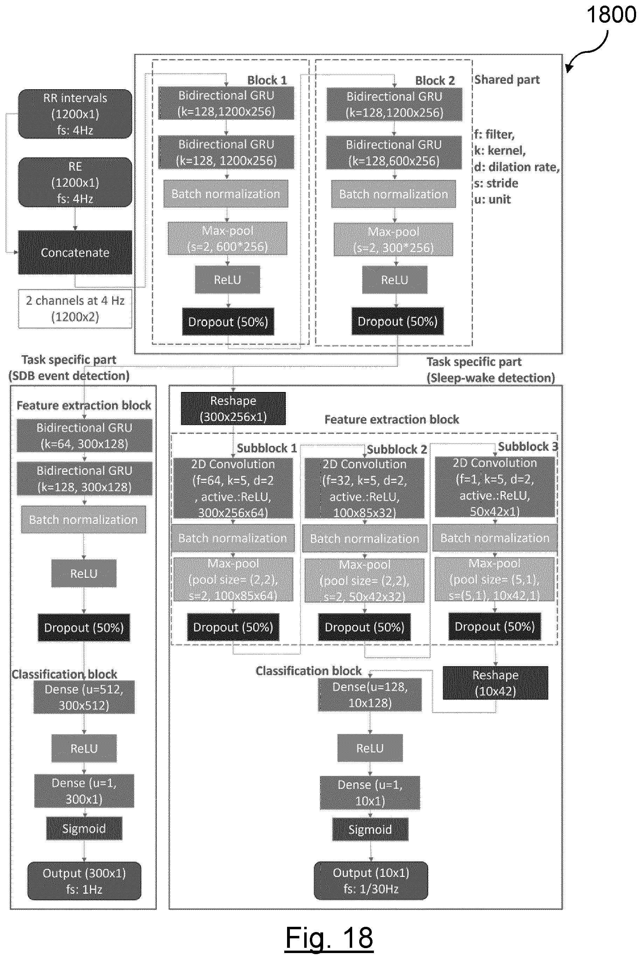

Fig. 7 illustrates an overview 700 of three exemplary architectures 702, 704 and 706 for the sleep-related event detection model according to embodiments of the present disclosure. It is to be understood that architectures 702, 704 and 706 are only examples and that also other architectures which build on the combination of cardiac and audio as input can achieve a similar improvement over the baseline model 300. The models 702, 704 and 706 may comprise one or more parts as described with respect to Fig. 18. In general, besides the technical advantages achieved by implementing models based on said combined input, adding audio as input has the advantage that corresponding audio signals can be relatively easy acquired via corresponding second sensor means such as microphones which are nowadays included in all different types of devices such as smartphones, smartwatches or other wearables. Compared to first sensor means which are used for capturing cardiac input (e.g., one or more cardiac signals) and are often in contact with the subject (e.g., a photoplethysmography or a chest-worn accelerometer), the second sensor means are often not in (direct) contact with the subject and/or are located near to the subject, which allows capturing audio recording signals from the environment of the subject. A sampling rate of the one or more cardiac signals may be different from a sampling rate of the one or more audio recording signals. In other words, the first and second sensor means may capture the corresponding data at different rates. A sampling rate of the cardiac signals may be higher/lower than the sampling rate of the audio recording signals.

Each model 702, 704 and 706 may be trained using the method 100 for training a sleep-related event detection model according to aspects of the present disclosure. Afterwards, each model 702, 704 and 706 may be used for providing the method 200 of sleep-related event detection according to aspects of the present disclosure.

Each model 702, 704 and 704 may comprise a shared part configured to generate one or more common features of the at least first and second sleep-related event using the one or more cardiac and/or audio recording signals. The at least two sleep-related event specific parts may comprise a first sleep-related event specific part configured to generate a detection result for the first sleep-related event and/or a second sleep-related event specific part configured to generate a detection result for the second sleep-related event. Each sleep-related event specific part may further be configured to receive the one or more common features and to generate the detection result of the sleep-related event based at least in part on the one or more common features. The one or more audio recording signals may serve as a surrogate for measured respiratory signals.

Model 702 of the first architecture illustrates an approach in which one or more audio recording signals and one or more cardiac signals (e.g., ECG signals) are captured by the corresponding first and second sensor means. By applying corresponding pre-processing, RR-interval series segments as well as EDR segments may be extracted from the collected ECG signals. By applying corresponding pre-processing, Mel Frequency Cepstral Coefficients (MFCCs) may be extracted from the one or more audio recording signals. In addition, or alternatively, spectrograms may be extracted from the one or more audio recording signals. Afterwards, a convolutional neural network (CNN) 702a may be applied to the audio recording signals (e.g., the extracted MFCCs and/or spectrograms) to obtain additional environmental information therefrom. The CCNs output (i.e., the additional environmental information) together with the one or more cardiac signals (e.g., RR interval series segments and/or EDR segments) may then be provided as input for the multi-task deep learning model to train the multi-task deep learning model to detect and generate corresponding detection results (e.g., a first sleep-related event like sleep-wake results such as a waking and sleep stage; and/or a second sleep-related event such as SDB events).

Model 704 of the second architecture illustrates an approach in which one or more audio recording signals and one or more cardiac signals (e.g., ECG signals) are captured by the corresponding first and second sensor means. By applying corresponding pre-processing, RR-interval series segments as well as EDR segments may be extracted from the collected ECG signals. By applying corresponding pre-processing, Mel Frequency Cepstral Coefficients (MFCCs) may be extracted from the one or more audio recording signals. In addition, or alternatively, spectrograms may be extracted from the one or more audio recording signals. In addition, the shared part 704a of the model 704 (e.g., a RNN) may only use the one or more cardiac signals (i.e., the shared part 704a is not using the one or more audio recording signals during training and inference). The model 704 may comprise a further part 704b configured to generate environmental features based on the one or more audio recording signals. As illustrated, the further part 704b may comprise a CRRN (i.e., a combination of a CNN and RNN as illustrated by "CNN+RNN"). The first sleep-related event specific part 704c (e.g., a CNN) may only use the common features generated by the shared part 704a (e.g., the RNN which only uses the one or more cardiac signals to generate the common features), to generate a detection result for the first sleep-related event (e.g., sleep-wake results). The second sleep-related event specific part 704d (e.g., a RNN) may be further configured to generate the detection result of the second sleep-related event (e.g., SBD results) based on the common features generated by the shared part 704a and the environmental features generated by the further part 704b.

Model 706 of the third architecture illustrates an approach in which one or more audio recording signals and one or more cardiac signals (e.g., ECG signals) are captured by the corresponding first and second sensor means. By applying corresponding pre-processing, RR-interval series segments as well as EDR segments may be extracted from the collected ECG signals. By applying corresponding pre-processing, Mel Frequency Cepstral Coefficients (MFCCs) may be extracted from the one or more audio recording signals. In addition, or alternatively, spectrograms may be extracted from the one or more audio recording signals. Other than models 702 and 704, the architecture of model 706 comprises a first part 706a configured to generate a detection result for the first sleep-related event (e.g., sleep-wake results) and a preliminary detection result for the second sleep-related event (e.g., ECG based SDB results) using the one or more cardiac signals. The first part 706a may comprise or be a multi-task model based on cardiac input. The model 706 may comprise a second part 706b configured to generate a preliminary detection result for the second sleep-related event (e.g., audio based SDB results) using the one or more audio recording signals. The second part 706b may be a single task model based on audio input. The model 706 may comprise a third part 706c configured to generate a (final) detection result for the second sleep-related event (e.g., final SDB results) based on the preliminary result(s) (e.g., ECG based SDB and/or audio based SDB results). The third part 706c may further be configured to generate a weighting factor. The third part 706c comprise be a deep-learning model trained to generate the weighting factor. Generating the (final) detection result for the second sleep-related event may further be based on the weighting factor. Training the deep-learning model may be done by applying binary labels to the preliminary results. A label "0" indicates that the preliminary detection result generated by the first part for the second sleep-related event using the one or more cardiac signals (e.g., the ECG based SDB result) is closer to the true sleep-related event (i.e., the ground truth) (e.g., the true SDB result). A label "1" indicates that the preliminary detection result generated by the second part for the second sleep-related event using the one or more audio recording signals (e.g., the audio based SDB result) is closer to the true sleep-related event (i.e., the ground truth) (e.g., the true SDB result). Each of the first 706a, second 706b and third part 706c may be trained independently. The labels may be established through a comparative analysis between ECG-based SDB results and audio based SDB results. The deep-learning model of the third part may output a binary classification (e.g., either 0 or 1) wherein "0" indicates that the ECG based SBD result is taken as final result and wherein "1" indicates that the audio based SBD result is taken as final result. Alternatively, a probability may be outputted if an integration of both preliminary detection results is desired. Both implementations (i.e., binary or probability) can be implemented using the following equation:

$$Final = 1st\ PDR * (1 - output) + 2nd\ PDR * output$$

wherein *final* represents the detection result for the second sleep-related event, *1st PDR* the preliminary result for the second sleep-related event using the one or more cardiac signals, *2nd PDR* the preliminary result for the second sleep-related event using the one or more audio recording signals and *output* the weighting factor generated by the third part 706c. It is to be understood that this is only a possible example and that depending on the applied labeling, the above-identified equation could also be adjusted (e.g., when labeling ECG based SBD results with 1 and audio based SDB results with 0). It should be noted that the models 702, 704 and 706 utilize additional input, namely the audio, to achieve an overall improvement with respect to the AHI estimation and SDB event detection, while at the same time not compromising or even slightly improving the sleep-wake detection.

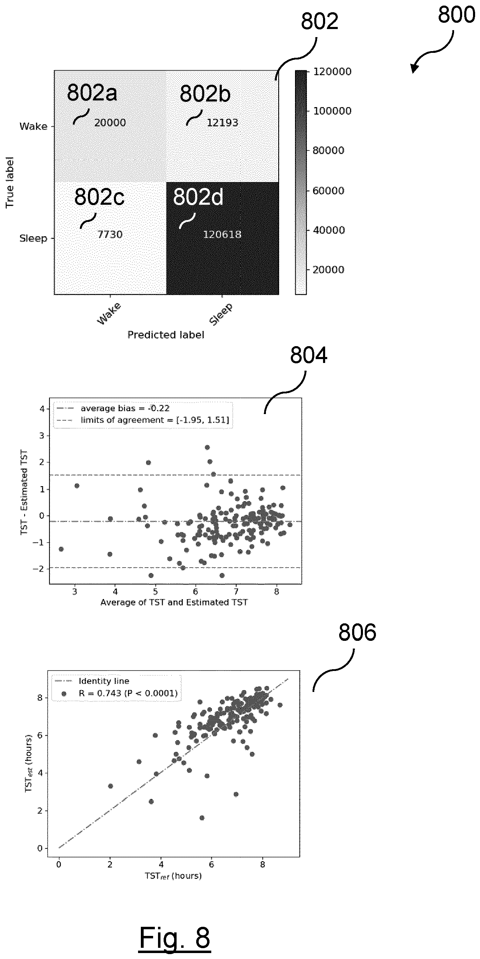

Fig. 8 illustrates first detection results 800 of a first sleep related event detection model 702. The first detection results 800 relate to sleep-wake detection results based on a combination of cardiac and audio input as explained with respect to Fig. 7. Confusion matrix 802 illustrates the performance of the model 702. Confusion matrix 802 comprises four fields, which relate to True Positive (TP) detections 802a, True negative (TN) detections 802d, False Positive (FP) detections 802b and False Negative (FN) detections 802c. TPs are instances that were correctly predicted as belonging to the positive class. TNs are instances that were correctly predicted as belonging to the positive class. FPs are instances that were incorrectly predicted as belonging to the positive class. FNs are instances that were incorrectly predicted as belonging to the negative class. As can be seen, the model 702 achieved a solid performance with respect to "sleep" detections. The model 702 detected from a total of 128,348 "sleep" samples, 120,618 samples correctly as "sleep" and only 7,730 wrongly as "wake". However, with respect to "wake" detections, the model 702 achieved a significant improvement compared to the performance of the baseline model 300. From 32,193 "wake" samples, the model 702 detected 20,000 correct and only 12,193 wrong. This implies that the model 702 was able to provide a good balance between a good sensitivity to "wake" detection without compromising too much of the "sleep" detection. From this improvement, one can follow that the baseline model 300 struggled most with finding a way to cope with the FP detections during the wake periods resulting in an unsatisfactory imbalance.

Bland-Altman plot 804 illustrates the total sleep time (TST) estimation results achieved by the model 702. The Bland-Altman plot displays the differences between two measurement methods (e.g., the actual TST and the estimated TST) on the y-axis (vertical axis) against the means/averages of the two methods on the x-axis (horizontal axis). The primary purpose of a Bland-Altman plot is to visualize the level of agreement or bias between the two measurement methods, helping to identify systematic differences, outliers, and the overall spread or variability of the differences. As can be seen, the average bias between the actual TST and the estimated TS of the model 702 is now only -0.22. In other words, this implies that the difference between the TST and the estimated TST is in average negative, i.e., the actual TST is shorter than the estimated TST.

Scatter plot 806 illustrates the estimated TST (in hours) compared to the actual/reference TST (in hours). The "identity line" serves as a reference line that represents a perfect positive linear correlation between the two variables (i.e., $TST_{est}$ and $TST_{ref}$). The Pearson correlation coefficient "R" is a measure of the linear relationship or correlation between the two. R takes values between -1 and 1 and helps to quantify the strength and direction of the linear relationship. R=1 implies a perfect positive linear correlation. In other words, as one variable increases, the other also increases in a linear fashion. R=-1 implies a perfect negative linear correlation. In other words, as one variable increases, the other decreases in a linear fashion. R0= implies no linear correlation. In other words, there is no linear relationship between the two variables. As can be seen, $TST_{est}$ and $TST_{ref}$ are positive linear correlated (see R= 0.743). Ideally, the predictions (visualized as the points) are close to the ideal line. As can be seen, compared to the performance of the baseline model 300, the points of model 702 are way closer to the ideal line. Accordingly, the performance of model 702 has improved over the performance of model 300.

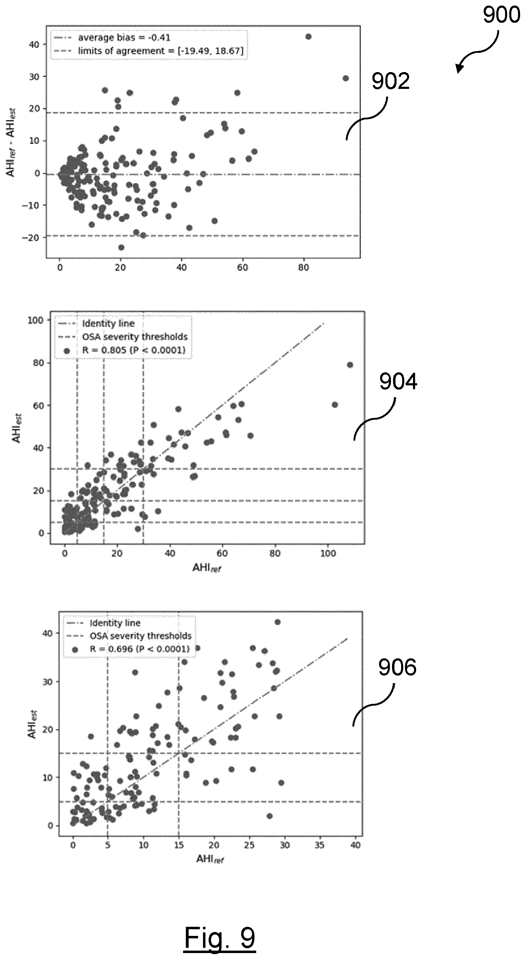

Fig. 9 illustrates second detection results 900 of the first sleep-related event detection model 702. The second detection results 900 relate to estimation of apnea-hypopnea index (AHI) based on a combination of cardiac and audio input as explained with respect to Fig. 7.

Bland-Altman plot 902 illustrates the AHI estimation results achieved by the model 702. The Bland-Altman plot displays the differences between two measurement methods (e.g., the actual/reference $AHI_{ref}$ and the estimated $AHI_{est}$) on the y-axis (vertical axis) against the means/averages of the two on the x-axis (horizontal axis). The primary purpose of a Bland-Altman plot is to visualize the level of agreement or bias between the two measurement methods, helping to identify systematic differences, outliers, and the overall spread or variability of the differences. As can be seen, average bias between the $AHI_{ref}$ and the $AHI_{est}$ is -0.41. In other words, this implies that the difference between the two is in average negative, i.e., the actual AHI is lower than the estimated AHI. However, more important is the observable change in the limits of agreement and corresponding data points lying within and without which implies that the model 702 is generally in a better agreement with the ground truth data than the baseline model 300.

Scatter plot 904 illustrates the estimated AHI compared to the actual/reference AHI. Scatter plot 904 illustrates the estimation results over the entire AHI range. The obstructive sleep apnea (OSA) severity thresholds are indicated by the dotted lines. The "identity line" serves as a reference line that represents a perfect positive linear correlation between the two variables (i.e., $AHI_{ref}$ and the $AHI_{est}$). The Pearson correlation coefficient "R" is a measure of the linear relationship or correlation between the two. R takes values between -1 and 1 and helps to quantify the strength and direction of the linear relationship. R=1 implies a perfect positive linear correlation. In other words, as one variable increases, the other also increases in a linear fashion. R=-1 implies a perfect negative linear correlation. In other words, as one variable increases, the other decreases in a linear fashion. R0= implies no linear correlation. In other words, there is no linear relationship between the two variables. As can be seen, $AHI_{ref}$ and $AHI_{est}$ are positive linear correlated (see R= 0.805). Ideally, the predictions (visualized as the points) are close to the ideal line. As can be seen, the points of the model 702 are closer to the identity line compared to the performance of the model 300. As a result, an improvement over the model 300 was achieved.

Scatter plot 906 illustrates the estimated AHI compared to the actual/reference AHI. Scatter plot 906 only comprises the estimations below the threshold of severe OSA (e.g., $AHI_{ref}<30$). The obstructive sleep apnea (OSA) severity thresholds are indicated by the dotted lines. The "identity line" serves as a reference line that represents a perfect positive linear correlation between the two variables (i.e., $AHI_{ref}$ and the $AHI_{est}$). As can be seen, $AHI_{ref}$ and $AHI_{est}$ are positive linear correlated (see R= 0.696). Ideally, the predictions (visualized as the points) are close to the ideal line. As can be seen, the points of the model 702 are more or less identically close to the identity line as the points of the model 300. Accordingly, the model 702 performed as well as the model 300.

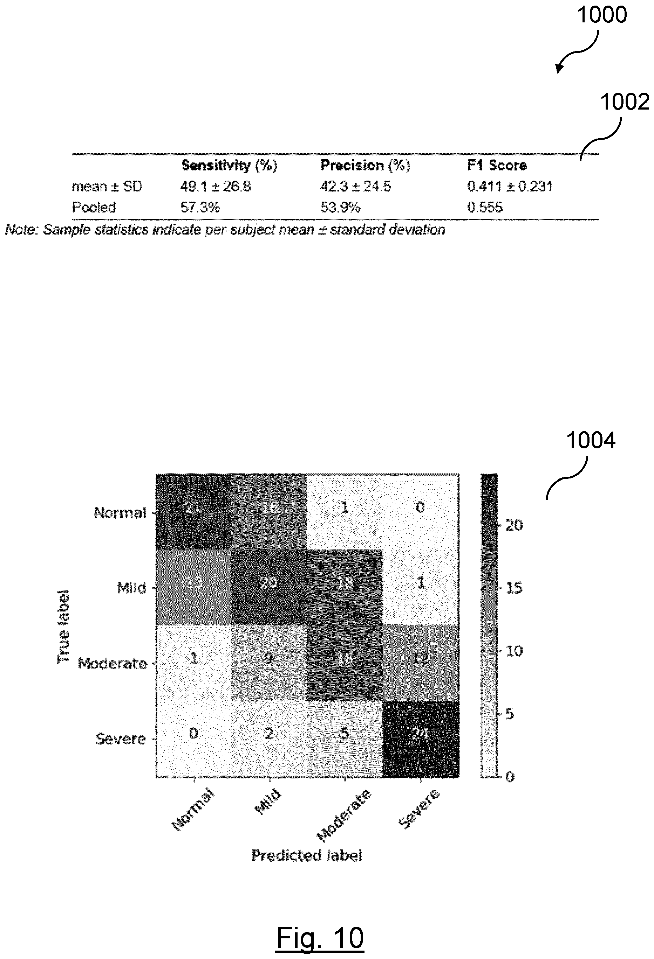

Fig. 10 illustrates third detection results 1000 of the first sleep-related event detection model 702. The third detection results 1000 relate to Sleep-disordered breathing (SBD) detection results based on a combination of cardiac and audio input as explained with respect to Fig. 7 and obstructive sleep apnea (OSA) severity classification results based on a combination of cardiac and audio input as explained with respect to Fig. 7.

The SBD detection results are illustrated in table 1002. The illustrated sample statistics indicate per-subject mean $\pm$ standard deviation (SD). The first column of table 1002 comprises the mean $\pm$ standard deviation (SD) sensitivity (%) of 49.1 $\pm$ 26.8 and the pooled sensitivity of 57.3. Sensitivity (or also called Recall or True Positive Rate) indicates the

rate between $\dfrac{TPs}{(TPs+FNs)}$. The second column of table 1002 comprises the mean $\pm$ SD precision (%) of 42.3 $\pm$ 24.5 and

the pooled precision of 53.9. The precision indicates the rate between $\dfrac{TPs}{(TPs+FPs)}$. The third column of table 1002 comprises the mean $\pm$ SD F1 Score of 0.4111 $\pm$ 0.231 and the pooled F1 Score of 0.555. The F1 Score indicates the rate

between $2 * \dfrac{(Precision*Sensitivity)}{(Precision+Sensitivity)}$. As can be seen, an improvement was achieved over the performance of model 300.

The OSA severity classification results are illustrated in confusion matrix 1004. The Confusion matrix illustrates the performance of the model 702 with respect to True Positive (TP) detections, True negative (TN) detections, False Positive (FP) detections and False Negative (FN) detections. The four severity classes the model had to distinguish between are "normal", "mild", "moderate" and "severe" OSA severity. As can be seen, the model 702 performed better than the model 300. In particular, the performance with respect to the classification of "severe" and "moderate" was significantly improved resulting in an overall improvement of the OSA severity classification performance. Furthermore, it can be observed that the results of model 702 comprise less outlier classifications (e.g., classifying "normal" as "severe") than the model 300, which indicates that model 702 is more robust than model 300.

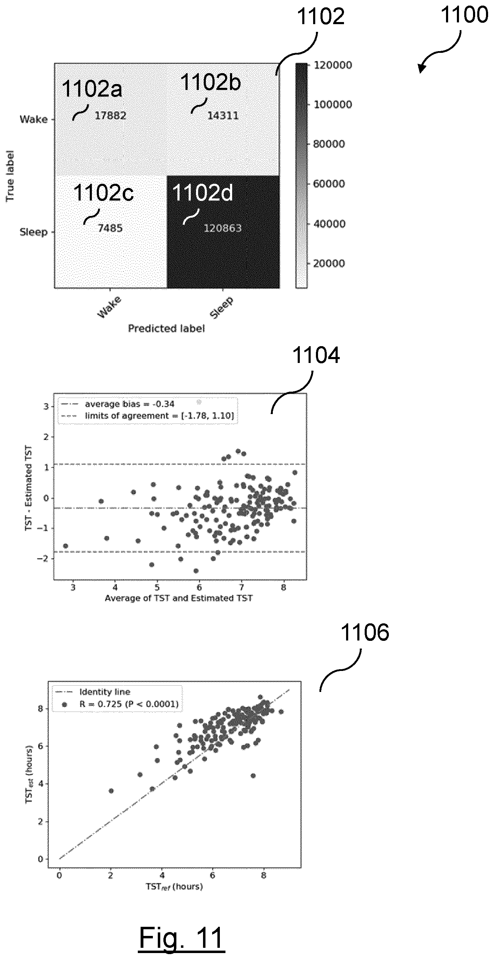

Fig. 11 illustrates first detection results 1100 of a second sleep related event detection model 704. The first detection results 1100 relate to sleep-wake detection results based on a combination of cardiac and audio input as explained with respect to Fig. 7. Confusion matrix 1102 illustrates the performance of the model 704. Confusion matrix 1102 comprises four fields, which relate to True Positive (TP) detections 1102a, True negative (TN) detections 1102d, False Positive (FP) detections 1102b and False Negative (FN) detections 1102c. TPs are instances that were correctly predicted as belonging to the positive class. TNs are instances that were correctly predicted as belonging to the positive class. FPs are instances that were incorrectly predicted as belonging to the positive class. FNs are instances that were incorrectly predicted as belonging to the negative class. As can be seen, the model 704 achieved a solid performance with respect to "sleep" detections. The model 704 detected from a total of 128348 "sleep" samples, 120,863 samples correctly as "sleep" and only 7,485 wrongly as "wake". However, with respect to "wake" detections, the model 704 achieved a significant improvement compared to the performance of the baseline model 300. From 32,193 "wake" samples, the model 704 detected 17,882 correct and only 14,311 wrong.

Bland-Altman plot 1104 illustrates the total sleep time (TST) estimation results achieved by the model 704. The Bland-Altman plot displays the differences between two measurement methods (e.g., the actual TST and the estimated TST) on the y-axis (vertical axis) against the means/averages of the two methods on the x-axis (horizontal axis). The primary purpose of a Bland-Altman plot is to visualize the level of agreement or bias between the two measurement methods, helping to identify systematic differences, outliers, and the overall spread or variability of the differences. As can be seen, the average bias between the actual TST and the estimated TS of the model 704 is now only -0.34. In other words, this implies that the difference between the TST and the estimated TST is in average negative, i.e., the actual TST is shorter than the estimated TST.

Scatter plot 1106 illustrates the estimated TST (in hours) compared to the actual/reference TST (in hours). The "identity line" serves as a reference line that represents a perfect positive linear correlation between the two variables (i.e., $TST_{est}$ and $TST_{ref}$). The Pearson correlation coefficient "R" is a measure of the linear relationship or correlation between the two. R takes values between -1 and 1 and helps to quantify the strength and direction of the linear relationship. R=1 implies a perfect positive linear correlation. In other words, as one variable increases, the other also increases in a linear fashion. R=-1 implies a perfect negative linear correlation. In other words, as one variable increases, the other decreases in a linear fashion. R0= implies no linear correlation. In other words, there is no linear relationship between the two variables. As can be seen, $TST_{est}$ and $TST_{ref}$ are positive linear correlated (see R= 0.725). Ideally, the predictions (visualized as the points) are close to the ideal line. As can be seen, compared to the performance of the baseline model 300, the points of model 704 are way closer to the ideal line. Accordingly, the performance of model 704 has improved over the performance of model 300.

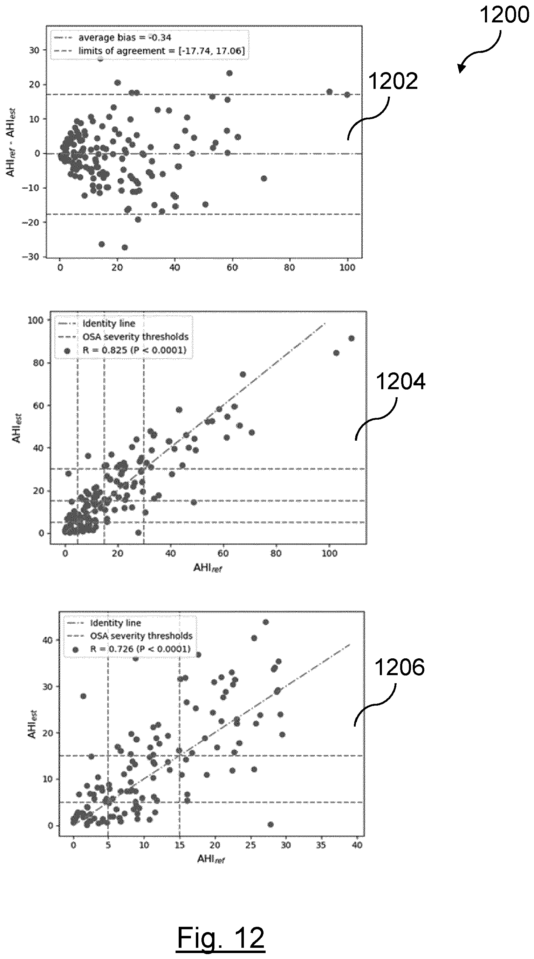

Fig. 12 illustrates second detection results 1200 of the second sleep-related event detection model 704. The second detection results 1200 relate to estimation of apnea-hypopnea index (AHI) based on a combination of cardiac and audio input as explained with respect to Fig. 7.

Bland-Altman plot 1202 illustrates the AHI estimation results achieved by the model 704. The Bland-Altman plot displays the differences between two measurement methods (e.g., the actual/reference $AHI_{ref}$ and the estimated $AHI_{est}$) on the y-axis (vertical axis) against the means/averages of the two on the x-axis (horizontal axis). The primary purpose of a Bland-Altman plot is to visualize the level of agreement or bias between the two measurement methods, helping to identify systematic differences, outliers, and the overall spread or variability of the differences. As can be seen, average bias between the $AHI_{ref}$ and the $AHI_{est}$ is -0.34. In other words, this implies that the difference between the two is in average negative, i.e., the actual AHI is lower than the estimated AHI.

Scatter plot 1204 illustrates the estimated AHI compared to the actual/reference AHI. Scatter plot 1204 illustrates the estimation results over the entire AHI range. The obstructive sleep apnea (OSA) severity thresholds are indicated by the dotted lines. The "identity line" serves as a reference line that represents a perfect positive linear correlation between the two variables (i.e., $AHI_{ref}$ and the $AHI_{est}$). The Pearson correlation coefficient "R" is a measure of the linear relationship or correlation between the two. R takes values between -1 and 1 and helps to quantify the strength and direction of the linear relationship. R=1 implies a perfect positive linear correlation. In other words, as one variable increases, the other also increases in a linear fashion. R=-1 implies a perfect negative linear correlation. In other words, as one variable increases, the other decreases in a linear fashion. R0= implies no linear correlation. In other words, there is no linear relationship between the two variables. As can be seen, $AHI_{ref}$ and $AHI_{est}$ are positive linear correlated (see R= 0.825). Ideally, the predictions (visualized as the points) are close to the ideal line. As can be seen, the points of the model 704 are closer to the identity line compared to the performance of the model 300. As a result, an improvement over the model 300 was achieved.

Scatter plot 1206 illustrates the estimated AHI compared to the actual/reference AHI. Scatter plot 1206 only comprises the estimations below the threshold of severe OSA (e.g., $AHI_{ref}$<30). The obstructive sleep apnea (OSA) severity thresholds are indicated by the dotted lines. The "identity line" serves as a reference line that represents a perfect positive linear correlation between the two variables (i.e., $AHI_{ref}$ and the $AHI_{est}$). As can be seen, $AHI_{ref}$ and $AHI_{est}$ are positive linear correlated (see R= 0.726). Ideally, the predictions (visualized as the points) are close to the ideal line. As can be seen, the points of the model 704 are closer to the identity line as the points of the model 300. Accordingly, the model 704 performed better than the model 300.

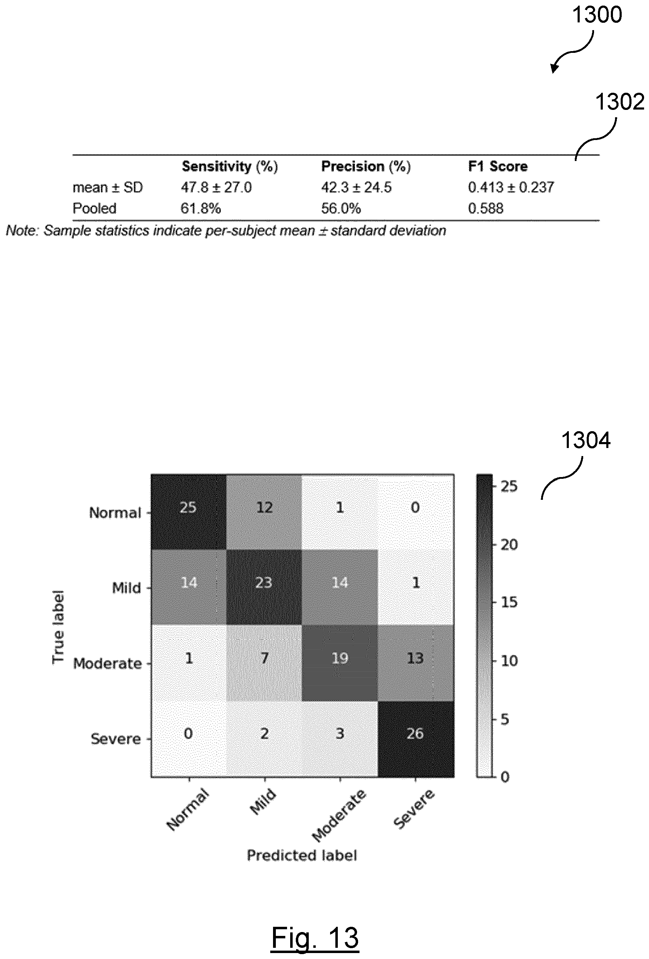

Fig. 13 illustrates third detection results 1300 of the second sleep-related event detection model 704. The third detection results 1300 relate to Sleep-disordered breathing (SBD) detection results based on a combination of cardiac and audio input as explained with respect to Fig. 7 and obstructive sleep apnea (OSA) severity classification results based on a combination of cardiac and audio input as explained with respect to Fig. 7.

The SBD detection results are illustrated in table 1302. The illustrated sample statistics indicate per-subject mean $\pm$ standard deviation (SD). The first column of table 1302 comprises the mean $\pm$ standard deviation (SD) sensitivity (%) of 47.8 $\pm$ 27.0 and the pooled sensitivity of 61.8. Sensitivity (or also called Recall or True Positive Rate) indicates the rate between $\dfrac{TPs}{(TPs+FNs)}$. The second column of table 1302 comprises the mean $\pm$ SD precision (%) of 42.3 $\pm$ 24.5 and the pooled precision of 56.0. The precision indicates the rate between $\dfrac{TPs}{(TPs+FPs)}$. The third column of table 1302 comprises the mean $\pm$ SD F1 Score of 0.413 $\pm$ 0.237 and the pooled F1 Score of 0.588. The F1 Score indicates the rate between $2 * \dfrac{(Precision*Sensitivity)}{(Precision+Sensitivity)}$. As can be seen, an improvement was achieved over the performance of model 300.

The OSA severity classification results are illustrated in confusion matrix 1304. The Confusion matrix illustrates the performance of the model 704 with respect to True Positive (TP) detections, True negative (TN) detections, False Positive (FP) detections and False Negative (FN) detections. The four severity classes the model had to distinguish between are "normal", "mild", "moderate" and "severe" OSA severity. As can be seen, the model 704 performed better than the model 300. In particular, the performance with respect to the classification of "severe" and "moderate" was improved. Furthermore, it can be overserved that the results of model 704 comprise less outlier classifications (e.g., classifying "normal" as "severe") than the model 300, which indicates that model 704 is more robust than model 300.

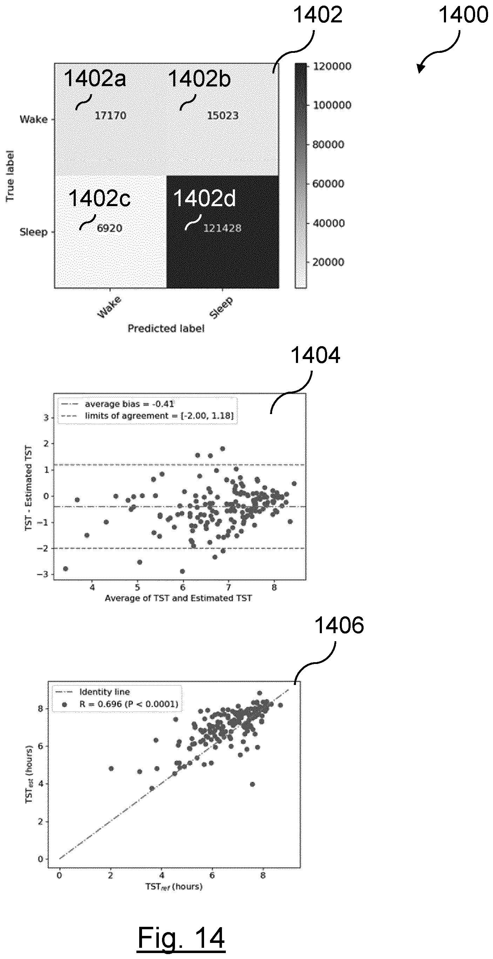

Fig. 14 illustrates first detection results 1400 of a third sleep related event detection model 706. The first detection results 1400 relate to sleep-wake detection results based on a combination of cardiac and audio input as explained with respect to Fig. 7. Confusion matrix 1402 illustrates the performance of the model 706. Confusion matrix 1402 comprises four fields, which relate to True Positive (TP) detections 1402a, True negative (TN) detections 1402d, False Positive (FP) detections 1402b and False Negative (FN) detections 1402c. TPs are instances that were correctly predicted as belonging to the positive class. TNs are instances that were correctly predicted as belonging to the positive class. FPs are instances that were correctly predicted as belonging to the positive class. FNs are instances that were incorrectly predicted as belonging to the negative class. As can be seen, the model 704 performed equally well compared to the model 300. With respect to "sleep" detections, the model 704 detected from a total of 128,348 "sleep" samples, 121,428 samples correctly as "sleep" and only 6,920 wrongly as "wake". With respect to "wake" detections, the model 704 detected from 32,193 "wake" samples 17,170 correct and 15,023 wrong.

Bland-Altman plot 1404 illustrates the total sleep time (TST) estimation results achieved by the model 706. The Bland-Altman plot displays the differences between two measurement methods (e.g., the actual TST and the estimated TST) on the y-axis (vertical axis) against the means/averages of the two methods on the x-axis (horizontal axis). The primary purpose of a Bland-Altman plot is to visualize the level of agreement or bias between the two measurement methods, helping to identify systematic differences, outliers, and the overall spread or variability of the differences. As can be seen, the average bias between the actual TST and the estimated TS of the model 706 is -0.41. In other words, this implies that the difference between the TST and the estimated TST is in average negative, i.e., the actual TST is shorter than the estimated TST.

Scatter plot 1406 illustrates the estimated TST (in hours) compared to the actual/reference TST (in hours). The "identity line" serves as a reference line that represents a perfect positive linear correlation between the two variables (i.e., $TST_{est}$ and $TST_{ref}$). The Pearson correlation coefficient "R" is a measure of the linear relationship or correlation between the two. R takes values between -1 and 1 and helps to quantify the strength and direction of the linear relationship. R=1 implies a perfect positive linear correlation. In other words, as one variable increases, the other also increases in a linear fashion. R=-1 implies a perfect negative linear correlation. In other words, as one variable increases, the other decreases in a linear fashion. R0= implies no linear correlation. In other words, there is no linear relationship between the two variables. As can be seen, $TST_{est}$ and $TST_{ref}$ are positive linear correlated (see R= 0.696).

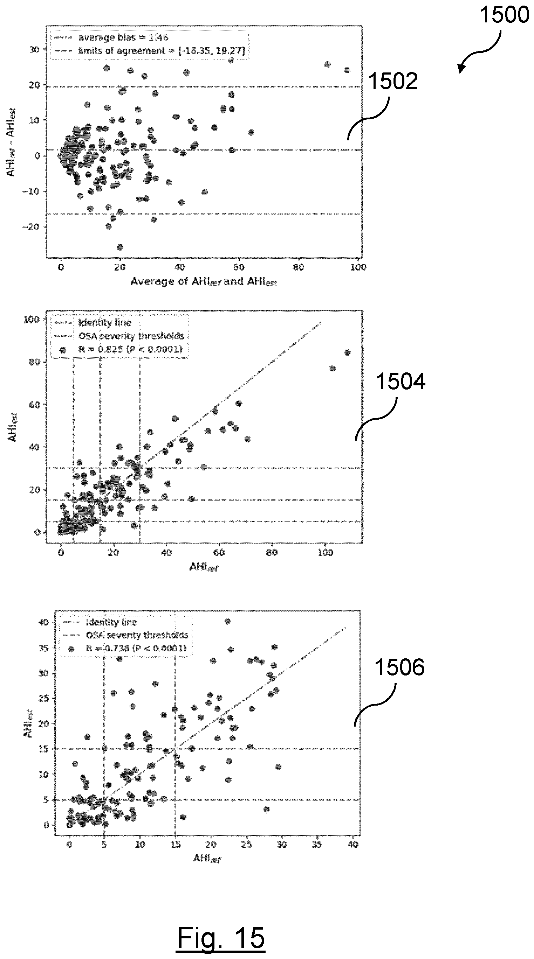

Fig. 15 illustrates second detection results 1500 of the third sleep-related event detection model 706. The second detection results 1500 relate to estimation of apnea-hypopnea index (AHI) based on a combination of cardiac and audio input as explained with respect to Fig. 7.

Bland-Altman plot 1502 illustrates the AHI estimation results achieved by the model 706. The Bland-Altman plot displays the differences between two measurement methods (e.g., the actual/reference $AHI_{ref}$ and the estimated $AHI_{est}$) on the y-axis (vertical axis) against the means/averages of the two on the x-axis (horizontal axis). The primary purpose of a Bland-Altman plot is to visualize the level of agreement or bias between the two measurement methods, helping to identify systematic differences, outliers, and the overall spread or variability of the differences. As can be seen, average bias between the $AHI_{ref}$ and the $AHI_{est}$ is 1.46. In other words, this implies that the difference between the two is in average positive, i.e., the actual AHI is higher than the estimated AHI.

Scatter plot 1504 illustrates the estimated AHI compared to the actual/reference AHI. Scatter plot 1504 illustrates the estimation results over the entire AHI range. The obstructive sleep apnea (OSA) severity thresholds are indicated by the dotted lines. The "identity line" serves as a reference line that represents a perfect positive linear correlation between the two variables (i.e., $AHI_{ref}$ and the $AHI_{est}$). The Pearson correlation coefficient "R" is a measure of the linear relationship or correlation between the two. R takes values between -1 and 1 and helps to quantify the strength and direction of the linear relationship. R=1 implies a perfect positive linear correlation. In other words, as one variable increases, the other also increases in a linear fashion. R=-1 implies a perfect negative linear correlation. In other words, as one variable increases, the other decreases in a linear fashion. R0= implies no linear correlation. In other words, there is no linear relationship between the two variables. As can be seen, $AHI_{ref}$ and $AHI_{est}$ are positive linear correlated (see R= 0.825). Ideally, the predictions (visualized as the points) are close to the ideal line. As can be seen, the points of the model 706 are closer to the identity line compared to the performance of the model 300. As a result, an improvement over the model 300 was achieved.

Scatter plot 1506 illustrates the estimated AHI compared to the actual/reference AHI. Scatter plot 1506 only comprises the estimations below the threshold of severe OSA (e.g., $AHI_{ref}<30$). The obstructive sleep apnea (OSA) severity thresholds are indicated by the dotted lines. The "identity line" serves as a reference line that represents a perfect positive linear correlation between the two variables (i.e., $AHI_{ref}$ and the $AHI_{est}$). As can be seen, $AHI_{ref}$ and $AHI_{est}$ are positive linear correlated (see R= 0.738). Ideally, the predictions (visualized as the points) are close to the ideal line. As can be seen, the points of the model 706 are closer to the identity line as the points of the model 300. Accordingly, the model 706 performed better than the model 300.

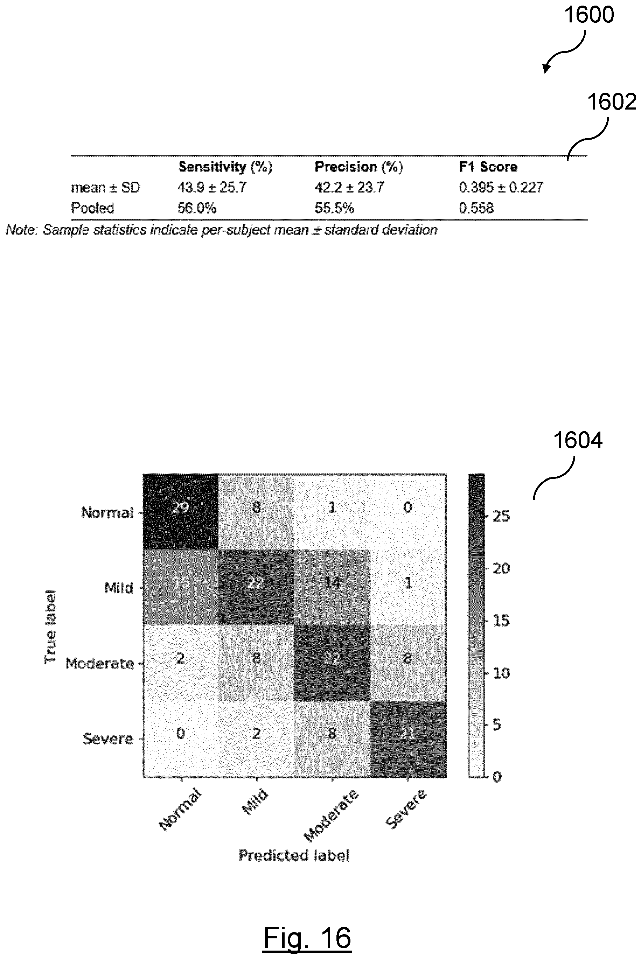

Fig. 16 illustrates third detection results 1600 of the third sleep-related event detection model 706. The third detection results 1600 relate to Sleep-disordered breathing (SBD) detection results based on a combination of cardiac and audio input as explained with respect to Fig. 7 and obstructive sleep apnea (OSA) severity classification results based on a combination of cardiac and audio input as explained with respect to Fig. 7.

The SBD detection results are illustrated in table 1602. The illustrated sample statistics indicate per-subject mean $\pm$ standard deviation (SD). The first column of table 1602 comprises the mean $\pm$ standard deviation (SD) sensitivity (%) of 43.9 $\pm$ 25.7 and the pooled sensitivity of 56.0. Sensitivity (or also called Recall or True Positive Rate) indicates the rate between $\dfrac{TPs}{(TPs+FNs)}$. The second column of table 1602 comprises the mean $\pm$ SD precision (%) of 42.2 $\pm$ 23.7 and the pooled precision of 55.5. The precision indicates the rate between $\dfrac{TPs}{(TPs+FPs)}$. The third column of table 1602 comprises the mean $\pm$ SD F1 Score of 0.395 $\pm$ 0.227 and the pooled F1 Score of 0.588. The F1 Score indicates the rate between $2 * \dfrac{(Precision*Sensitivity)}{(Precision+Sensitivity)}$. As can be seen, an improvement was achieved over the performance of model 300.

The OSA severity classification results are illustrated in confusion matrix 1604. The Confusion matrix illustrates the performance of the model 706 with respect to True Positive (TP) detections, True negative (TN) detections, False Positive (FP) detections and False Negative (FN) detections. The four severity classes the model had to distinguish between are "normal", "mild", "moderate" and "severe" OSA severity. As can be seen, the model 706 performed better than the model 300. In particular, the performance with respect to the classification of "severe" and "moderate" was improved. Furthermore, it can be overserved that the results of model 764 comprise less outlier classifications (e.g., classifying "normal" as "severe") than the model 300, which indicates that model 706 is more robust than model 300.

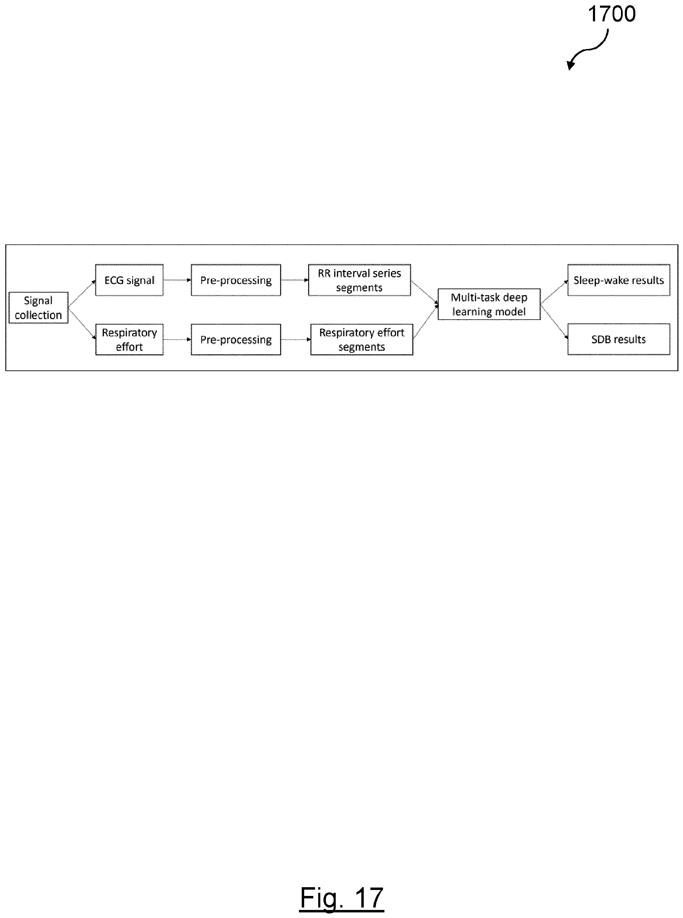

Fig. 17 illustrates an overview 1700 of a multi-task model in accordance with embodiments of the present disclosure. A possible architecture of the multi-task model is explained with respect to Fig. 18. The multi-task model may use cardiac (e.g., ECG) and respiratory (e.g., respiratory effort (RE)) inputs (or other signals such as one or more audio recording signals, which serve as a surrogate for measured respiratory signals). The multi-task model may be trained to simultaneously generate detection results for a first sleep-related event (e.g., SDB events from which SDB event intervals such as start and end or start and duration may be derived) and a second sleep-related event. For example, the second sleep-related event may relate to sleep stages such as sleep or wake for each epoch of an (overnight) recording. Epochs may relate to non-overlapping segments of a certain duration (e.g., 30 seconds). Sleep stages may comprise wake, N1, N2, N3, and REM or any combination thereof such as combined N1 and N2, combined N1-N3 (also called non-REM). Instead of categorical output, the model may also output probabilities (e.g. adding up to 1) for each output class.

Implementing the multi-task model 1800 according to aspects of the present disclosure may provide one or more of the following technical advantages compared to the known approaches comprising single-task models:
Efficient Resource Utilization: The multi-task model 1800 can share parameters and computation across different tasks. This leads to more efficient resource utilization, as one can train a single model to perform multiple tasks instead of training separate models for each task. This is particularly important in situations where computational resources are limited.

Improved Generalization: The multi-task model 1800 can generalize better because it can leverage the common features and patterns shared across different tasks. This can lead to better performance on each task compared to training separate single-task models, especially when data for each individual task is limited.

Regularization: Multi-task learning acts as a form of regularization. By learning multiple tasks simultaneously, the model 1800 is forced to discover more general and robust features. This helps prevent overfitting and enhances the model's ability to adapt to new data and tasks.

Transfer Learning: The multi-task model 1800 can facilitate transfer learning. Once a multi-task model is trained on a set of tasks, it can be fine-tuned for a specific task by freezing some layers and training only the task-specific layers. This transfer learning can be very effective when you have a small amount of task-specific data.

Inter-Task Relationships: The multi-task model 1800 can capture relationships between tasks. If there are dependencies or correlations between tasks, the multi-task model 1800 can explicitly model these relationships, leading to improved performance on all tasks. For example, in signal processing (e.g., processing one or more audio recording signals and/or cardiac signals), the multi-task model 1800 can simultaneously learn tasks like SBD event detection and sleep-staging detection wherein the performance regarding each task benefit from "trained" knowledge about shared common features.

Simplified Deployment: Using the multi-task model 1800 can simplify deployment in production systems. Instead of managing and serving multiple single-task models, you can deploy a single multi-task model 1800, reducing operational complexity.

Few-Shot Learning: The multi-task model can facilitate few-shot learning. Because it is designed to learn multiple tasks, it is more adaptable to new tasks (e.g., by adding further specific parts) with minimal data.

Regularized Feature Extraction: The multi-task model 1800 can provide useful features that can be used for downstream tasks. These features are learned in a multi-task context and can serve as a strong feature extractor, making it easier to build other models or systems (i.e., technical applications) on top of them.

Data Efficiency: Multi-task learning can make more efficient use of the available data. When training separate single-task models, each model may require a substantial amount of data to achieve good performance. In contrast, the multi-task model 1800 can benefit from a shared dataset, potentially reducing the need for a large dataset for each task.

Time Efficiency: Training a single multi-task model can be faster than training multiple single-task models, as you save time on setting up, running, and maintaining separate experiments.

In the following a method for implementing (i.e., data collection, preprocessing, post-processing and/or training) the multi-task model is explained.

In a first step ("signal collection") signals (e.g., ECG and RE signals may be collected from one or more subjects.

In a step 2 ("preprocessing") the collected signals may be divided into segments (e.g., ECG into RR interval series segments and RE into RE segments). The step of preprocessing as explained in the following may also be used for the preprocessing as mentioned in the other embodiment, in particular Fig. 3 and Fig. 7.

For the cardiac signals (e.g., ECG), R-peaks detection may initially be performed. For example, using an algorithm based on nonlinear transformation and simple peak-finding strategy. Subsequently, a post-processing algorithm may be employed to precisely localize the QRS complexes and eliminate artifacts. Additionally, an algorithm may be applied to address ectopic beats. Periods containing artifacts or ectopic beats may be marked with a value of 0 for exclusion. The resulting RR intervals may then be subjected to linear interpolation and resampled at a frequency of 4 Hz (e.g., a sample rate of the one or more cardiac signals). In case a different cardiac sensor is used, this methodological step should be changed to make it adequate to the characteristics of the input signal, provided that the output corresponds to interbeat interval time series (or equivalent), describing the time distance between consecutive heart beats. For example, using PPG as input, one would perform a pulse detection (e.g., by detecting the peak, the trough, or the inflection point, etc) instead of QRS peak detection. The time difference between the location of each pulse will thus be essentially equivalent to the RR interval time series described in this embodiment.

The RE signals may also be resampled (e.g., to the same or different sample rate of the one or more cardiac signals). For example, the RE signals may be resampled to the same sample rate (e.g., 4H) to ensure consistency with the RR interval series segments. Resampling may comprise eliminating high frequency noise (e.g., >2Hz). A high-pass filter (e.g., with a certain cut-off frequency such as 0.05 Hz) may be utilized to remove low frequency noise from the RE signals. Instead of collecting the RE-signals with a dedicated sensor, it may also be possible obtain the respiratory effort by means of suitable EDR techniques, whereby respiratory effort is indirectly measured by the changes in electrode impedance due to respiratory movements (e.g., inflation and deflation of the chest which leads to changes in the distance between the electors) which in turn translate to changes on the peak amplitude and in the area under the QRS complexes. While such signals may be less presentative of respiratory effort (e.g., compared to collecting RE signals with a dedicated sensor), they may still provide a sufficient adequate representation of respiratory changes so that they can be used for sleep-related event detection (e.g., SDB event detection and sleep staging). Alternatively or additionally, the RE may be derived from other sensors such as PPG, a thoracic respiratory belt, bed sensors, doppler radar or other suitable sensing means.

Afterwards, the cardiac and RE signals may be segmented into segments of a predefined duration (e.g., 5 minutes). The segments may overlap for a predefined duration (e.g., 2 minutes). As a result, a plurality of segments may be obtained each comprising a corresponding pair of an RR interval and a RE time series). Afterwards, each segment may be normalized (e.g., using soft min-max normalization with the minimum and maximum values set to the 5th and 95th percentiles). The resulting RR and RE data of each segment may be stacked to form an input vector (e.g., bivariate) of a certain shape comprising the number of samples and channels (e.g., 1200 samples x 2 channels). For scoring of SBD events, they may be mapped into segments of the same predefined duration (e.g., 5 minutes) with the same overlap duration (e.g., 2 minutes). Within such a segment, one sample of a one-second period may be used and set to "1" to indicate an SDB event of any type (e.g., obstructive, central, mixed apneas, hypopneas etc.) occurring during that duration or set to "0" if normal breathing was performed during that duration. Performing this procedure for all available data may result in a vector of a certain shape comprising the number of samples per segment and the corresponding label (e.g., for 300 samples per segment and each sample labeled as either "0" or "1", a vector of shape of 300x1 is generated). For the sleep-stages, annotations of sleep-stages may be mapped into segments with the same predefined duration and the same predefined overlap duration. However, a different sampling rate corresponding to the epoch duration of the sleep stages may be used for the sleep-stages segments (e.g., 1/30 Hz). Accordingly, the duration of the epoch may represent the duration of a sample. For example, 30 seconds. Each sample that corresponds to the sleep stage "Wake" may then be assigned with a label of "0" and each sample corresponding to a sleep stage (e.g., N1, N2, N3, REM or any combination thereof) may be assigned a label of "1". Accordingly, each segment may be represented with a sleep label vector of a certain shape comprising the number of epochs within the segment as well as the corresponding label (e.g., a label of shape 10x1, for 10 epochs per segment each labeled either "0" or "1"). Both labels combined (e.g., the SDB events and the Wake/Sleep stages) may represent the targeted first and second sleep-related events the multi-task model is trained to detect. It may be preferable to remove segments occurring during periods at the start and end of the recording (e.g., due to lights being turned off/on).

It is to be understood that this only is an example of a possible pre-processing/segmentation strategy. Other strategies are also applicable such as implementing the model without any segmentation and instead use the complete recording(s) at input.

In a third step ("training), the model may be trained using the data (e.g., preprocessed as explained in the previous step) to afterwards be able to provide simultaneous detection results for a first and second sleep-related event (e.g., sleep-wake results and SDB results).

Training may be performed using a corresponding optimizer with a corresponding learning rate, weight decay and batch size. For example, Adam optimizer with a learning rate of 0.001 and a weight decay of 0.0001, and a batch size of 128 may be used. Model initialization may be performed using a corresponding initializer. For example, model initialization may be performed using the Xavier uniform initializer. In case of imbalance of the samples with respect to the labels (e.g., imbalance between apnea/hypopnea events and normal periods), sample weighting may be implemented. For example, a weight of 10 may be assigned to apnea/hypopnea events and a weight of 1 to normal breathing periods.

For mitigating the risk of overfitting, kernel regularization, dropout and/or early stopping may be used. Early stopping may be configured such that training is terminated when no decrease in the validation loss was observed in a predefined number of epochs (e.g., 10 epochs).

In a third step ("postprocessing"), the model output may be further processed so that a meaningful evaluation of the model performance can be performed.

For sleep-wake detection, the model may output a value between 0 to 1. To obtain a binary classification, a threshold may be selected for determining whether an output represents a "sleep" (e.g., a positive class, labeled 1) or a "wake" (e.g., negative class, labeled 0) classification. Selecting the threshold may be done for each cross-validation iteration on a validation set. Selecting the threshold may be based on a threshold value yielding the best F1 score for the sleep versus wake classification for each corresponding cross-validation iteration. This threshold may then be used, on the same cross-validation iteration, to obtain a binary classification on segments of recordings of a testing set. Finally, performance may be evaluated by comparing the classification for each epoch (e.g., six 30-second epochs) of a middle part of a segment. The middle part may refer to the predefined duration minus the predefined overlapping duration. For example, in a 5 minute segment, the outer 2 minutes which overlap with neighboring segments may be discarded, such that the middle part contains the three remaining middle minutes. Results from the recording (e.g., the whole night) may be combined to estimate the TST.

For SDB event detection, evaluation may be performed on the middle parts of each segment (e.g., also the three middle minutes). Each segment may be transformed into events for the evaluation. Transforming may comprise assigning a sample (e.g., a one-second sample) to an event if the model output for the SDB event detection surpasses a threshold. Samples scored as part of an SDB event during a period detected as Wake by the assessment above may then be assigned to 'normal breathing' periods. The threshold used to decide whether a sample was part of an SDB event may be automatically determined by maximizing the F1 score for event detection on the validation set of each cross-validation iteration.

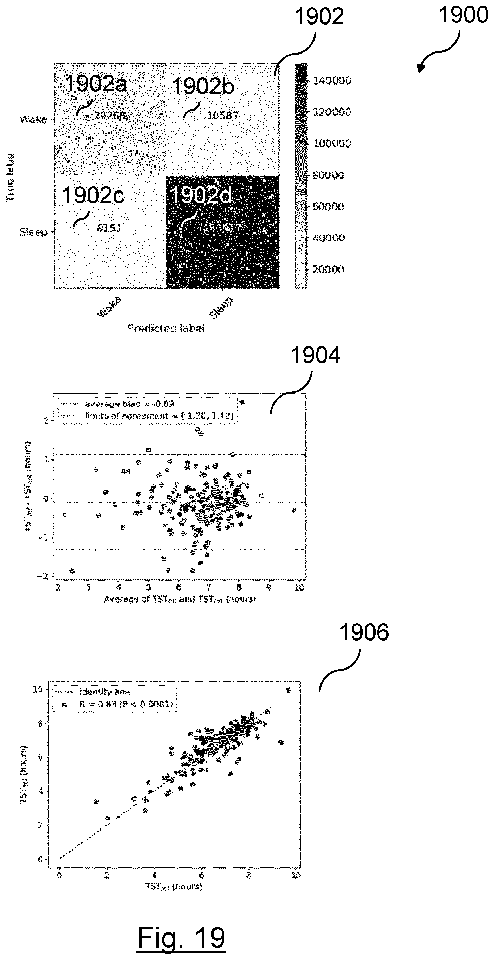

The results of the performed evaluation are described with respect to Figs. 19-21. The model was evaluated by a four-fold cross-validation approach to effectively leverage the available dataset for model evaluation and training. The division of the dataset into four folds was done through a stratified random split method, designed to maintain a balanced distribution of subjects across all levels of OSA severity. Initially, all subjects were categorized into four distinct OSA severity groups based on their AHI values from the annotation: normal (AHI < 5), mild OSA (5 < AHI < 15), moderate OSA (15 < AHI < 30), and severe OSA (AHI > 30). Subsequently, subjects within each severity group were randomly assigned to four subsets, and one subset was chosen from each group to form one fold of the cross-validation procedure. During each iteration, one fold was designated as the testing set, while the remaining three folds were merged and further partitioned into training and validation sets. To create the training and validation sets, the three folds were combined and subsequently divided into the four OSA severity groups. 75% of the subjects from each group were taken for training, and the remaining 25% for validation, configuration of the training, validation, and testing sets in the first iteration of the four-fold cross-validation process. This process was repeated for each cross-validation iteration, with a different fold designated as the testing set in each round, while the training and validation sets were assembled as described. In each iteration of the cross-validation process, the model was fitted using the training set, while the validation set was utilized for early stopping and to determine the optimal decision threshold. The results obtained on each recording of the testing set for each cross-validation iteration were finally combined to assess the overall performance for all subjects in the dataset.

Fig. 18 illustrates an exemplary implementation of a multi-task model 1800 for detecting sleep-related events in accordance with embodiments of the present disclosure. The illustrated architecture for the multi-task model 1800 may be used for the multi-task deep learning models as explained with respect to Fig. 7, wherein the audio recording signals may serve as a surrogate for the respiratory signals as used for the model 1800.

The multi-task deep learning model 1800 architecture may consist of three main components. The first main component may be a shared part for both tasks (i.e., detection of a first and a second sleep-related event) which is configured to generate one or more common features for the first (e.g., SBD event detection) and second sleep-related event (e.g., sleep staging such as sleep-wake detection). The shared part is designed to learn common latent representations relevant to both tasks. It may comprise two blocks, each block consisted of two layers of bidirectional gated recurrent units (GRU), a batch normalization layer, a max-pool layer, an activation layer utilizing the rectified linear unit (ReLU) activation function, and a dropout layer. Obviously different architectures are feasible, provided that they are able to learn the relations within each, and between each signal, that are relevant for both tasks.

The second main component may be a first sleep-related event specific part configured to generate a detection result for the first sleep-related event (e.g., a task specific part for SDB event detection. The task-specific part for SDB event detection may consist of a feature extraction block and a classification block. The feature extraction block may encompass two layers of bidirectional GRUs, a batch normalization layer, an activation layer using the ReLU activation function, and a dropout layer. Subsequently, the classification block may consist of a dense-connected layer employing the ReLU activation function and another dense-connected layer utilizing the sigmoid activation function to generate the output. This SDB-specific part may provide an output at 1 Hz, allowing for each second of the output to be classified as belonging to (or not) an SDB interval. In contrast with other methods described in literature, where classification is performed on an epoch-per-epoch basis (e.g. on epochs of 30 seconds, or 1 minute, or 5 minutes) and the output indicates only whether there is (or not) at least one SDB event occurring during that epoch, the presented approach allow to determine the start and the duration (or the end) of each SDB event with a 1-second accuracy. This SDB-specific part may also be configured to output with different resolutions to provide finer accuracies for interval detection (e.g., at 2 Hz, to allow for a 0.5 second precision, or lower frequencies such as 0.5 Hz, or even lower if such accuracies are not required). Regardless of the output frequency, the presented approach has the technical advantage over the prior art, which only provides a per epoch classification, that the presented approach allows to separate SDB events of arbitrary length (e.g. 10 seconds) that occur close to each other (e.g. within 5 seconds) and which would have been otherwise "grouped" in the same epoch, for which only an indication of whether there is "any" event would be available, instead of an indication that in that period two SDB events occur. Accordingly, if an SDB event would start in one epoch and end in the next (or even, further away) epoch, an epoch-based algorithm would provide an indication of 2 or more consecutive epochs as having an SDB event, whereas the present approach is able to precisely indicate that a single SDB event occurred.

The third main component may be a second sleep-related event specific part configured to generate a detection result for the second sleep-related event (e.g., a task specific part for sleep-wake detection). The task-specific part for sleep-wake detection may include a feature extraction block combined with three subblocks. Each subblock may be composed of a 2-dimensional convolution layer with ReLU activation, a batch normalization layer, a max-pool layer, and a dropout layer. Additionally, two reshape layers may be incorporated at the beginning and end of the feature to adjust the shape for input and output. The classification block for sleep-wake detection can mirror that of event detection, but with a different output resolution.

The model 1800 enables the training of different tasks simultaneously even for outputs with different shapes. Besides leveraging the commonalities and dependencies of both tasks, it is more efficiency than using two separate single-task models. It can also be extended to more than one tasks (i.e., more than two sleep-related events) by adding a corresponding sleep-related event specific part for each additional sleep-related event detection ask.

Fig. 19 illustrates first detection results 1900 of the multi-task model 1800 for detecting sleep-related events in accordance with embodiments of the present disclosure. The results 1900 relate to the sleep-wake detection. The multi-task model 1800 detected from a total of 159,068 "sleep" samples, 150,917 samples correctly as "sleep" and only 8,151 wrongly as "wake". From 39,855 "wake" samples, the multi-task model 1800 detected 29268 correct and only 10587 wrong. The multi-task model 1800 achieved a significant improvement compared to the performance of the baseline model 300.

Sleep-wake detection achieved an F1 score of 0.942 (0.936 $\pm$ 0.055 per subject) and a Cohen's kappa of 0.70 (0.66 $\pm$ 0.14 per subject) for the classification of all 30-second epochs into sleep and wake in comparison with scorings from PSG. The confusion matrix 1902 illustrating the TP predictions 1902a, the TN predictions 1902d, the FP predictions 1902b and the FN predictions 1902c was obtained after aggregating all epochs from all testing recordings. Bland-Altman plot 1904 and the scatter plot 106 illustrate the TST estimation. With a Spearman's correlation coefficient of R = 0.83 (P < 0.0001) between the reference TST ($TST_{ref}$) and the estimated TST ($TST_{est}$), the classifier slightly overestimated TST by 0.09 hours, with 95% limits of agreement of [-1.30, 1.21] hours.

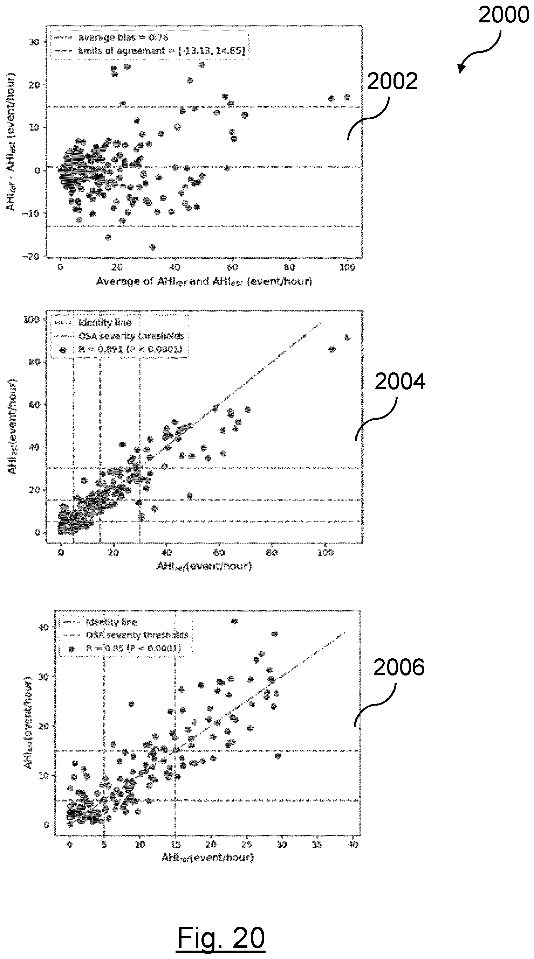

Fig. 20 illustrates second detection results 2000 of the multi-task model 1800 for detecting sleep-related events in accordance with embodiments of the present disclosure. The results 2000 relate to the AHI estimation.

The multi-task model 1800 achieved a Spearman's correlation coefficient between $AHI_{ref}$ (reference, obtained from manual scoring of the PSG) and $AHI_{est}$ (estimated with the presented method by dividing the total number of detected SDB events by the estimated total sleep time, obtained by summing the duration - in hours - of all epochs classified as sleep) of 0.891 (P<0.0001), with a small underestimation bias of 0.76 events/hour, and 95% limits of agreement of [-13.13, 14.65] event/hour. These values can be derived from the Bland-Altman plot 2002 and the scatter plot 2004 with the AHI estimation results, for both the complete AHI range. For the range of AHI below the threshold of severe OSA (AHI<30), the model 1800 achieved a Spearman's correlation coefficient of 0.850 (P<0.0001) as can be seen from scatter plot 2006.

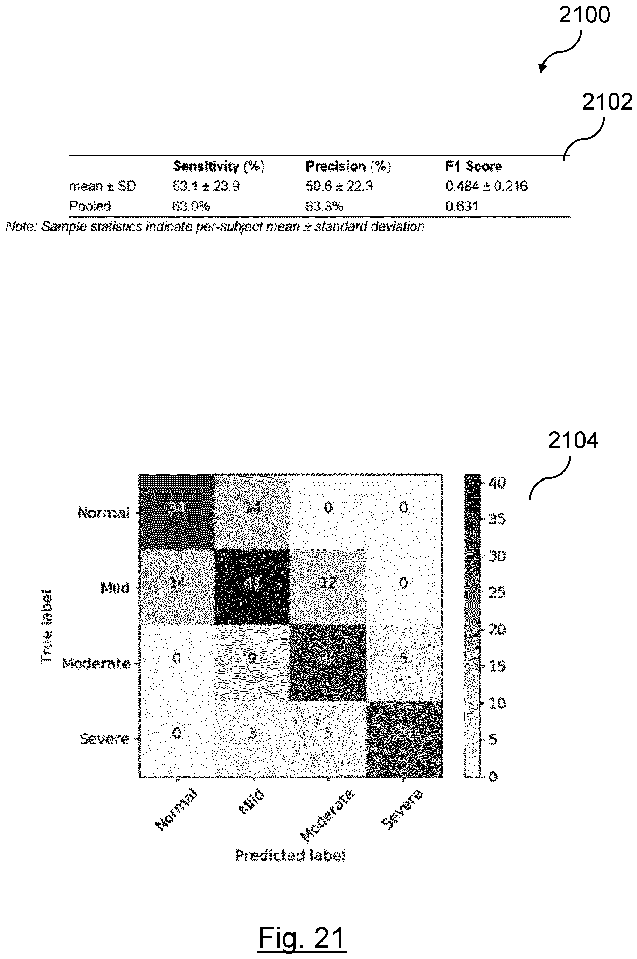

Fig. 21 illustrates third detection results 2100 of the multi-task model 1800 for detecting sleep-related events in accordance with embodiments of the present disclosure. The results 2100 relate to SDB event detection results as indicated by table 2102 and relate to OSA severity classification results as indicated by confusion matrix 2104.

Table 2102 presents the SBD event detection results in terms of mean and standard deviation per subject as well as the aggregated (pooled) outcomes from events from all recording. The model achieved an F1 score of 0.631 regarding the overall event detection performance.

As can be seen from the confusion matrix 2104, the model 1800 using the classical thresholds for OSA severity classification (i.e., normal (AHI < 5), mild OSA (5 < AHI < 15), moderate OSA (15 < AHI < 30), and severe OSA (AHI > 30), achieved an accuracy of 68.7% and a kappa of 0.58.

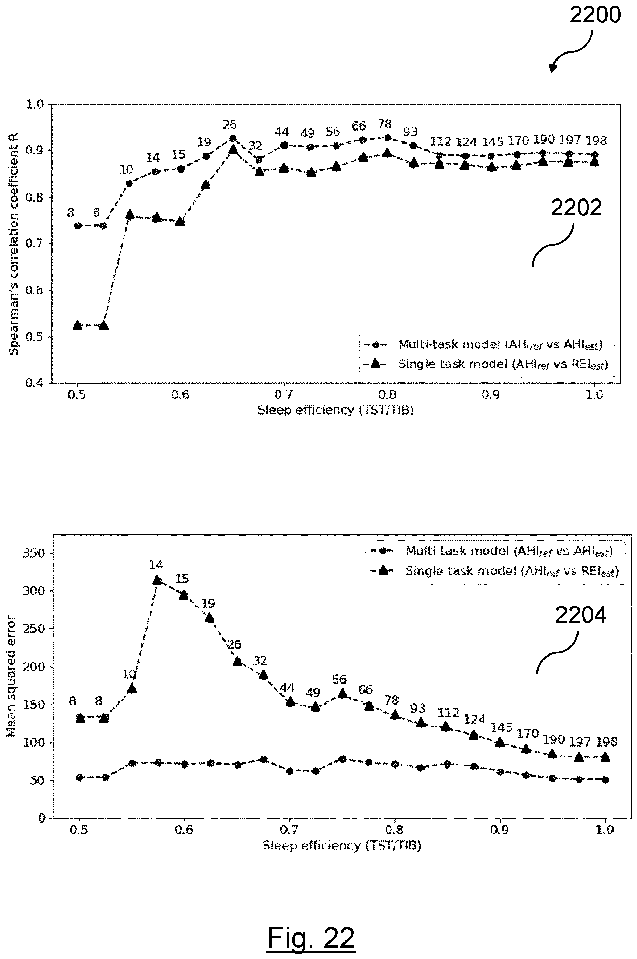

Fig. 22 illustrates a performance comparison between the multi-task model 1800 and a single task model. In addition to the multi-task model 1800, a separate single task model was trained to allow a direct comparison with the multi-task model 1800 for AHI estimation.

Diagram 2202 illustrates the Spearman's correlation coefficients for AHI estimation obtained with the multi-task model, which includes sleep-wake detection ($AHI_{ref}$ versus $AHI_{est}$), and with the single-task model, which does not include sleep-wake detection ($AHI_{ref}$ versus estimated respiratory event index or $REI_{est}$ obtained by dividing the number of detected SDB events by total time in bed (TIB)), for a varying threshold of maximum sleep efficiency (as scored from PSG) as a function of sleep efficiency. Each data point in the diagram 2202 represents the coefficient calculated for subjects with sleep efficiency lower than or equal to the corresponding value. Additionally, the number above each point indicates the total number of subjects included in the respective analysis. TIB stands for total time in bed.

Diagram 2204 illustrates the mean squared error (MSE) for the above described AHI estimations obtained with the multi-task model and with the single-task model as a function of sleep efficiency. Each data point in the diagram 2204 represents the MSE calculated from subjects with sleep efficiency lower than or equal to the corresponding value. Additionally, the number above each point indicates the total number of subjects included in the respective analysis. TIB stands for total time in bed.

As can be seen, the performance of the multi-task model 1800 is almost always superior to that of the single-task model, in particular for patients with a sleep efficiency below 65%. The performance converges as the sample increases to include subjects with higher sleep efficiency (more than half of the subjects had a sleep efficiency over 80%).

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments of the present disclosure may be implemented on a computer system. The computer system may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system may comprise any circuit or combination of circuits. In one embodiment, the computer system may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), or any other type of processor or processing circuit. Other types of circuits that may be included in the computer system may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system may include one or more storage devices, which may include one or more memory elements suitable to the particular application, such as a main memory in the form of random-access memory (RAM), one or more hard drives, and/or one or more drives that handle removable media such as compact disks (CD), flash memory cards, digital video disk (DVD), and the like. The computer system may also include a display device, one or more speakers, and a keyboard and/or controller, which can include a mouse, trackball, touch screen, voice-recognition device, or any other device that permits a system user to input information into and receive information from the computer system.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the present disclosure can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the present disclosure comprise a data carrier having electronically readable control signals, which are capable of cooperating with a programmable computer system, such that one of the methods described herein is performed.

Generally, embodiments of the present disclosure can be implemented as a computer program product with a program code, the program code being operative for performing one of the methods when the computer program product runs on a computer. The program code may, for example, be stored on a machine-readable carrier.

Other embodiments comprise the computer program for performing one of the methods described herein, stored on a machine-readable carrier.

In other words, an embodiment of the present disclosure is, therefore, a computer program having a program code for performing one of the methods described herein, when the computer program runs on a computer.

A further embodiment of the present disclosure is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitory. A further embodiment of the present disclosure is an apparatus as described herein comprising a processor and the storage medium.

A further embodiment of the present disclosure is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein. A further embodiment comprises a computer having installed thereon the computer program for performing one of the methods described herein.

A further embodiment according to the present disclosure comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

**Claims**

1. A computer-implemented method of training a sleep-related event detection model, wherein the method comprises: providing, as input, a set of training data samples, wherein each training data sample comprises:

   one or more cardiac signals representative of a cardiac parameter of a subject;
   one or more audio recording signals representative of an environmental sound of the subject; and
   at least a first and second sleep-related event of the subject associated with the one or more cardiac and audio recording signals of the subject; and
   training the sleep-related event detection model using the input.

2. A method of sleep-related event detection of a subject, wherein the method comprises:

   providing a trained sleep-related event detection model trained in accordance with the method of claim 1;
   providing, as input to the sleep-related event detection model, one or more cardiac signals representative of a cardiac parameter of a subject and one or more audio recording signals representative of an environmental sound of the subject;
   detecting, by the sleep-related event detection model, at least a first and/or second sleep-related event of the subject based at least in part on the input to generate a detection result; and

providing the detection result.

3. The method of any one of the preceding claims, wherein the sleep-related event detection model comprises:

a shared part configured to generate one or more common features of the at least first and second sleep-related event using the one or more cardiac and/or audio recording signals; and
at least two sleep-related event specific parts comprising:

a first sleep-related event specific part configured to generate a detection result for the first sleep-related event; and
a second sleep-related event specific part configured to generate a detection result for the second sleep-related event.

4. The method of the preceding claim, wherein each sleep-related event specific part is further configured to receive the one or more common features and to generate the detection result of the sleep-related event based at least in part on the one or more common features.

5. The method of any one of the claims 3-4, wherein method comprises:

applying, before providing the input, a convolutional neural network, CNN, on the one or more audio recording signals to obtain additional environmental information; and
providing, as part of the input, the additional environmental information.

6. The method of any one of the claims 3 and 4, wherein the shared part is using only the one or more cardiac signals;

wherein the sleep-related event detection model comprises a further part configured to generate environmental features based on the one or more audio recordings; and
wherein the second sleep-related event specific part is further configured to generate the detection result of the second sleep-related event based on the environmental features.

7. The method of any one of the claims 1-2, wherein the sleep-related detection model comprises:

a first part configured to generate a detection result for the first sleep-related event and a preliminary detection result for the second sleep-related event using the one or more cardiac signals;
a second part configured to generate a preliminary detection result for the second sleep-related event using the one or more audio recording signals; and
a third part configured to generate a detection result for the second sleep-related event based on the preliminary detection results.

8. The method of claim 7, wherein the third part is further configured to generate a weighting factor; and
wherein generating the detection result for the second sleep-related event is further based on the weighting factor.

9. The method of any one of the preceding claims, wherein the first sleep-related event comprises one or more sleep stages, such as a waking stage and a sleep stage; and/or
wherein the second sleep-related event comprises a sleep disorder breathing, SBD, event.

10. The method of any one of the preceding claims, wherein the one or more cardiac signals include electrocardiography, ECG, signals, in particular ECG-derived respiration, EDR, signals and/or signals of respiratory effort; and/or
wherein the one or more audio recording signals comprise Mel Frequency Cepstral Coefficients, MFCCs, extracted from the one or more audio recording signals and/or spectrograms extracted from the one or more audio recording signals.

11. The method of the preceding claim, wherein the one or more cardiac signals are captured using a first sensor means, in particular wherein the first sensor means is in contact with the subject, in particular a photoplethysmography or a chest-worn accelerometer; and/or
wherein the one or more audio recording signals are captured using a second sensor means such as a microphone, a smartphone and/or a smartwatch, in particular wherein the second sensor means is not in contact with the subject and/or is located near to the subject.

12. The method of any one of the preceding claims, wherein the one or more audio recording signals serve as a surrogate for measured respiratory signals of the subject; and/or wherein a sampling rate of the one or more cardiac signals is different from a sampling rate of the one or more audio recording signals.

13. A data-processing device comprising means for performing the method of any one of the claims 1-12.

14. A computer program or a computer-readable medium having stored thereon a computer program, the computer program comprising instructions which, when the program is executed by a computer and/or a computer network, cause the computer and/or the computer network to carry out the method of any one of the claims 1-12.

15. A data structure comprising a trained sleep-related event detection model trained using the method of any one of the claims 1 and 3-12.

100

Providing as input, a set of training data samples, wherein each training data sample comprises one or more cardiac signals, one or more audio recording signals and at least a first and second sleep-related event associated with the one or more cardiac and audio recording signals.
102

Training a sleep-related event detection model using the input.
104

Fig. 1

200

Providing a trained sleep-related event detection model.
202

Providing, as input to the sleep-related event detection model, one or more cardiac signals and one or more audio recording signals.
204

Detecting, by the sleep-related event detection model, at least a first and/or second sleep-related event based at least in part on the input to generate a detection result.
206

Providing the detection result.
208

Fig. 2

300

Fig. 3

402

400

402a
17170

402b
15023

402c
6920

402d
121428

Wake

Sleep

True label

Wake          Sleep
Predicted label

120000

100000

80000

60000

40000

20000

404

- - - average bias = -0.41
- - - limits of agreement = [-2.00, 1.18]

TST - Estimated TST

Average of TST and Estimated TST

406

- - - Identity line
- R = 0.696 (P < 0.0001)

TST$_{est}$ (hours)

TST$_{ref}$ (hours)

<u>Fig. 4</u>

Fig. 5

600

602

| | Sensitivity (%) | Precision (%) | F1 Score |
|---|---|---|---|
| mean ± SD | 46.2 ± 26.5 | 39.9 ± 24.6 | 0.387 ± 0.224 |
| Pooled | 56.6% | 52.0% | 0.542 |

Note: Sample statistics indicate per-subject mean ± standard deviation

604

Fig. 6

700

702

ECG signal → Pre-processing → RR interval series segments / EDR segments → Multi-task deep learning model → Sleep-wake results / SDB results

Audio → Pre-processing → MFCC segments → CNN

704

Modified multi-task deep learning model

704a

704c

ECG signal → Pre-processing → RR interval series segments / EDR segments → RNN → CNN → Sleep-wake results

Audio → Pre-processing → MFCCs segments → CNN+RNN → RNN → SDB results

704b

704d

Multi-task deep learning model

706

706a

ECG signal → Pre-processing → RR interval series segments / EDR segments → Multi-task deep learning model → Sleep-wake results / ECG based SDB results

Audio → Pre-processing → MFCC segments → Single-task deep learning model → Audio based SDB results

Label=0: ECG SDB result is closer to true SDB result
Label=1: Audio SDB result is closer to true SDB result

Training target

706b

706c

A deep-learning model to decide weight for the two SDB results → Output → ECG SDB results*(1-output) + Audio SDB results*output → Final SDB results

Fig. 7

Fig. 8

Fig. 9

1000

1002

|  | Sensitivity (%) | Precision (%) | F1 Score |
|---|---|---|---|
| mean ± SD | 49.1 ± 26.8 | 42.3 ± 24.5 | 0.411 ± 0.231 |
| Pooled | 57.3% | 53.9% | 0.555 |

*Note: Sample statistics indicate per-subject mean ± standard deviation*

1004

Fig. 10

1102                    1100

1102a          1102b
  17882          14311

Wake

True label

1102c          1102d
  7485           120863

Sleep

Wake          Sleep

Predicted label

120000

100000

80000

60000

40000

20000

1104

average bias = -0.34
limits of agreement = (-1.78, 1.10)

TST - Estimated TST

Average of TST and Estimated TST

1106

Identity line
R = 0.725 (P < 0.0001)

$TST_{est}$ (hours)

$TST_{ref}$ (hours)

<u>Fig. 11</u>

Fig. 12

1300

1302

| | Sensitivity (%) | Precision (%) | F1 Score |
|---|---|---|---|
| mean ± SD | 47.8 ± 27.0 | 42.3 ± 24.5 | 0.413 ± 0.237 |
| Pooled | 61.8% | 56.0% | 0.588 |

*Note: Sample statistics indicate per-subject mean ± standard deviation*

1304

Fig. 13

Fig. 14

Fig. 15

1600

1602

| | Sensitivity (%) | Precision (%) | F1 Score |
|---|---|---|---|
| mean ± SD | 43.9 ± 25.7 | 42.2 ± 23.7 | 0.395 ± 0.227 |
| Pooled | 56.0% | 55.5% | 0.558 |

*Note: Sample statistics indicate per-subject mean ± standard deviation*

1604

Fig. 16

1700

Fig. 17

1800

**Fig. 18**

Fig. 19

Fig. 20

2100

2102

| | Sensitivity (%) | Precision (%) | F1 Score |
|---|---|---|---|
| mean ± SD | 53.1 ± 23.9 | 50.6 ± 22.3 | 0.484 ± 0.216 |
| Pooled | 63.0% | 63.3% | 0.631 |

*Note: Sample statistics indicate per-subject mean ± standard deviation*

2104

Fig. 21

Fig. 22

Europäisches
Patentamt

European
Patent Office

Office européen
des brevets

# EUROPEAN SEARCH REPORT

**Application Number**

**EP 23 20 4570**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ARSLAN RECEP SINAN ET AL: "Sleep disorder and apnea events detection framework with high performance using two-tier learning model design", PEERJ COMPUTER SCIENCE, vol. 9, 29 September 2023 (2023-09-29), page e1554, XP093139037, ISSN: 2376-5992, DOI: 10.7717/peerj-cs.1554 Retrieved from the Internet: URL:https://www.ncbi.nlm.nih.gov/pmc/artic les/PMC10557519/pdf/peerj-cs-09-1554.pdf> | 1,2, 13-15 | INV. A61B5/0205 A61B5/024 A61B5/352 A61B5/00 |
| Y | * the whole document * | 3-12 | |
| Y | LIN XU ET AL: "Contactless sleep apnea detection in snoring signals using hybrid deep neural networks targeted for embedded hardware platform with real-time applications", BIOMEDICAL SIGNAL PROCESSING AND CONTROL, vol. 77, 20 May 2022 (2022-05-20), page 103765, XP093139748, NL ISSN: 1746-8094, DOI: 10.1016/j.bspc.2022.103765 * the whole document * | 3-12 | |
| X | AZADEH YADOLLAHI ET AL: "Sleep apnea monitoring and diagnosis based on pulse oximetery and tracheal sound signals", MEDICAL & BIOLOGICAL ENGINEERING & COMPUTING, SPRINGER, BERLIN, DE, vol. 48, no. 11, 24 August 2010 (2010-08-24), pages 1087-1097, XP019835568, ISSN: 1741-0444 * figure 1 * | 1-15 | |

**TECHNICAL FIELDS SEARCHED (IPC)**

A61B

-/--

The present search report has been drawn up for all claims

| Place of search | Date of completion of the search | Examiner |
|---|---|---|
| The Hague | 13 March 2024 | Lommel, André |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

# EUROPEAN SEARCH REPORT

Application Number

EP 23 20 4570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2023 0107460 A (UNIV KWANGWOON IND ACAD COLLAB [KR]) 17 July 2023 (2023-07-17) * paragraphs [0013], [0021] – [0026]; figures 1-3 * | 1-15 | |
| X | PRONIEWSKA KLAUDIA ET AL: "Classification of sleep disordered breathing in the evaluation of acoustic sound in correlation with the ECG signal", THE INSTITUTE OF ELECTRICAL AND ELECTRONICS ENGINEERS, INC. (IEEE) CONFERENCE PROCEEDINGS, 1 September 2014 (2014-09-01), pages 153-156, XP093140495, Piscataway Retrieved from the Internet: URL:https://ieeexplore.ieee.org/stampPDF/g etPDF.jsp?tp=&arnumber=7043002&ref=aHR0cHM 6Ly9pZWVleHBsb3JlLmllZWUub3JnL2RvY3VtZW50L zcwNDMwMDI=> * the whole document * | 1-15 | |

**TECHNICAL FIELDS SEARCHED (IPC)**

The present search report has been drawn up for all claims

| Place of search | Date of completion of the search | Examiner |
|---|---|---|
| The Hague | 13 March 2024 | Lommel, André |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 2 of 2**

## ANNEX TO THE EUROPEAN SEARCH REPORT
## ON EUROPEAN PATENT APPLICATION NO.

EP 23 20 4570

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2024

| Patent document cited in search report | Publication date | Patent family member(s) | Publication date |
|---|---|---|---|
| KR 20230107460 A | 17-07-2023 | NONE | |

EPO FORM P0459